# NEW EUROPEAN PATENT SPECIFICATION

(11) **EP 2 825 643 B2**
(45) Date of publication and mention of the opposition decision: **09.07.2025**
(45) Mention of the grant of the patent: 03.11.2021
(21) Application number: 13728877.5
(22) Date of filing: 24.05.2013
(51) Int. Cl.: C12N 9/26

(54) **VARIANT ALPHA AMYLASES WITH ENHANCED ACTIVITY ON STARCH POLYMERS**
VARIANTE ALPHA-AMYLASEN MIT ERHÖHTER WIRKUNG AUF STÄRKEPOLYMERE
VARIANTS D'ALPHA-AMYLASES AYANT UNE ACTIVITÉ ACCRUE SUR DES POLYMÈRES D'AMIDON

(30) Priority: 08.06.2012 US 201261657501 P
(43) Date of publication of application: 21.01.2015
(60) Divisional application: 21205366.4
(73) Proprietor: Danisco US Inc., Palo Alto, California 94304 (US)
(72) Inventor: BOTT, Richard R., Palo Alto California 94304 (US); CASCAO-PEREIRA, Luis G., Palo Alto California 94304 (US); ESTELL, David A., Palo Alto California 94304 (US); KOLKMAN, Marc, Palo Alto California 94304 (US); WILDES, David E., Palo Alto California 94304 (US)
(74) Representative: Mewburn Ellis LLP
(86) International application number: PCT/US2013/042702
(87) International publication number: WO 2014/007921

(56) References cited:
- WO-A1-2011/080352
- WO-A2-02/092797
- WO-A2-2009/149130
- WO-A2-2013/001087
- WO-A2-2013/057143
- US-A1- 2011 033 882
- US-B2- 7 166 453
- US-B2- 7 166 453
- US-B2- 7 498 158
- I. MATSUI ET AL: "Improved Activity and Modulated Action Pattern Obtained by Random Mutagenesis at the Fourth - Loop Involved in Substrate Binding to the Catalytic ( / )8-Barrel Domain of Barley -Amylase 1", JOURNAL OF BIOLOGICAL CHEMISTRY, vol. 272, no. 36, 5 September 1997 (1997-09-05), US, pages 22456 - 22463, XP055226298, ISSN: 0021-9258, DOI: 10.1074/jbc.272.36.22456
- TINE E. GOTTSCHALK ET AL: "Specificity Modulation of Barley [alpha]-Amylase through Biased Random Mutagenesis Involving a Conserved Tripeptide in [beta] -> [alpha] Loop 7 of the Catalytic ([beta]/[alpha]) 8 -Barrel Domain +", BIOCHEMISTRY, vol. 40, no. 43, 1 October 2001 (2001-10-01), US, pages 12844 - 12854, XP055226300, ISSN: 0006-2960, DOI: 10.1021/bi0108608
- HARUHIDE MORI ET AL: "Barley [alpha]-amylase Met53 situated at the high-affinity subsite -2 belongs to a substrate binding motif in the [beta]->[alpha] loop 2 of the catalytic ([beta]/[alpha])8-barrel and is critical for activity and substrate specificity", EUROPEAN JOURNAL OF BIOCHEMISTRY, vol. 269, no. 22, 25 October 2002 (2002-10-25), GB, pages 5377 - 5390, XP055226303, ISSN: 0014-2956, DOI: 10.1046/j.1432-1033.2002.03185.x
- I. MATSUI ET AL: "Improved Activity and Modulated Action Pattern Obtained by Random Mutagenesis at the Fourth - Loop Involved in Substrate Binding to the Catalytic ( / )8-Barrel Domain of Barley -Amylase 1", JOURNAL OF BIOLOGICAL CHEMISTRY, vol. 272, no. 36, 5 September 1997 (1997-09-05), US, pages 22456 - 22463, XP055226298, ISSN: 0021-9258, DOI: 10.1074/jbc.272.36.22456
- TINE E. GOTTSCHALK ET AL: "Specificity Modulation of Barley [alpha]-Amylase through Biased Random Mutagenesis Involving a Conserved Tripeptide in [beta] -> [alpha] Loop 7 of the Catalytic ([beta]/[alpha]) 8 -Barrel Domain +", BIOCHEMISTRY, vol. 40, no. 43, 1 October 2001 (2001-10-01), US, pages 12844 - 12854, XP055226300, ISSN: 0006-2960, DOI: 10.1021/bi0108608
- HARUHIDE MORI ET AL: "Barley [alpha]-amylase Met53 situated at the high-affinity subsite -2 belongs to a substrate binding motif in the [beta]->[alpha] loop 2 of the catalytic ([beta]/[alpha])8-barrel and is critical for activity and substrate specificity", EUROPEAN JOURNAL OF BIOCHEMISTRY, vol. 269, no. 22, 25 October 2002 (2002-10-25), GB, pages 5377 - 5390, XP055226303, ISSN: 0014-2956, DOI: 10.1046/j.1432-1033.2002.03185.x
- DECLERCK N ET AL: "Probing structural determinants specifying high thermostability in Bacillus licheniformis alpha-amylase", JOURNAL OF MOLECULAR BIOLOGY, ACADEMIC PRESS, UNITED KINGDOM, vol. 301, no. 4, 25 August 2000 (2000-08-25), pages 1041 - 1057, XP004461752, ISSN: 0022-2836, DOI: 10.1006/JMBI.2000.4025
- DATABASE Geneseq [online] 1 September 2011 (2011-09-01), "Cytophaga sp. calcium-sensitive alpha-amylase, SEQ ID 30.", retrieved from EBI accession no. GSP:AZK99874 Database accession no. AZK99874

## Description

### TECHNICAL FIELD

Described are compositions and methods relating to variant α-amylase enzymes for use in industrial processes, such as liquefaction of starch. The α-amylase variants have increased specific activity allowing the more rapidly reduction of peak viscosity during liquefaction processes.

### BACKGROUND

α-amylases are used for a variety of industrial and commercial processes, including starch liquefaction, textile desizing, food preparation, laundry cleaning and dishwashing. In such applications, α-amylases can breakdown starch to release smaller carbohydrates. However, starch bundles can be resistant to α-amylase hydrolysis because the organized starch polymers exclude the enzymes. As a result, using an α-amylase with increased specific activity only marginally improves starch hydrolysis because it does not address the problem of accessibility. The need exists for α-amylases that are more effective at accessing the starch polymers in starch bundles.

Matsui & Svensson, J. Biol. Chem., 272 (36), 22456 - 22463 (1997) describe barley α-amylases that have been mutated by local random mutagenesis to have improved activity toward amylose. Gottschalk et al., Biochem., 40 (43), 12844 - 12854 (2001) describe barley α-amylases that have been mutated by a biased random mutagenesis strategy to have modified substrate specificity and product profiles. Mori et al., Eur. J. Biochem., 269 (22), 5377 - 5390 (2002) describes a series of mutant α-amylases that contain mutations at residue Met53, which is situated at the high-affinity subsite -2 in barley α-amylase 1.

US 7,166,453 B2 describes methods of producing variant polypeptides having non-maltogenic exoamylase activity, the method comprising introducing an amino acid substitution into a parent polypeptide having non-maltogenic exoamylase activity, the amino acid substitution being selected from the group consisting of: G69P, A141P, G223A, A268P, G313P, S399P and G400P, with reference to the position numbering of a *Pseudomonas saccharophilia* exoamylase.

### SUMMARY

The invention provides methods for producing variant α-amylase polypeptides, as defined by the claims. Aspects and embodiments of the invention are summarized in the following separately-numbered paragraphs 1-14.
1. In one aspect, a method for producing a variant α-amylase polypeptide is provided according to claim 1.
6. In some embodiments of the method of any of the preceding paragraphs, the variant further comprises wild-type amino acid residues at one or more positions corresponding to N88, A252, A253, N308, A316, S357, T400, R402, and D403, referring to SEQ ID NO: 4 for numbering.
7. In some embodiments of the method of any of the preceding paragraphs, the variant further comprises wild-type amino acid residues at one or more positions corresponding to N4, G5, T38, N93, G94, I95, Q96, V97, Y230, G255, V315, P319, A322, L354, T355, R356, G359, Y396, A397, Y398, G399, and Q401 in SEQ ID NO: 4, referring to SEQ ID NO: 4 for numbering.
8. In some embodiments of the method of any of the preceding paragraphs, the variant further comprises N at position 88, A at position 252, A at position 253, N at position 308, A at position 316, S at position 357, T at position 400, R at position 402, or D at position 403, referring to SEQ ID NO: 4 for numbering. 4.
9. In some embodiments of the method of any of the preceding paragraphs, the variant further comprises N at position 4, G at position 5, T at position 38, N at position 93, G at position 94, I at position 95, Q at position 96, V at position 97, Y at position 230, G at position 255, V at position 315, P at position 319, A at position 322, L at position 354, T at position 355, R at position 356, G at position 359, Y at position 396, A at position 397, Y at position 398, G at position 399, and Q at position 401, referring to SEQ ID NO: 4 for numbering.
10. In some embodiments of the method of any of the preceding paragraphs, relative starch-binding is determined using cyclodextrin.
12. In some embodiments of the method of any of the preceding claims, the parent has at least 90% amino acid sequence identity to SEQ ID NO:.
13. In some embodiments of the method of any of the preceding claims, the variant exhibits improved starch liquefaction, starch saccharification, or cleaning performance compared to the parent.
15. The present disclosure also sets out a number of variant polypeptides and compositions as discussed herein below. The invention is however defined by the accompanying claims.
16. Described herein is a variant α-amylase polypeptide produced by the method of the preceding paragraphs.
17. Also described herein is a variant of a parent Family 13 α-amylase polypeptide, comprising a mutation in the starch-binding groove; wherein the starch-binding groove is formed by amino acid residues in the α-helix preceding the first β-stand in the A domain, the loop between the sixth α-helix and the seventh β-strand in the A domain, the loop between the seventh α-helix and the eighth β-strand in the A domain, and the loop connecting the A domain and the C domain; wherein the mutation alters the binding of starch to the variant α-amylase polypeptide compared to the parental α-amylase polypeptide.
18. Described herein is the variant α-amylase polypeptide of paragraph 17, wherein the starch-binding groove corresponds to amino acid residues 1-6, 36, 38, 91-97, 224-226, 249-257, 278-282, 309-320, 354-359, 391, and 395-402, referring to SEQ ID NO: 2 for numbering, amino acid residues 1-6, 37, 39, 92-98, 227-229, 252-260, 281-285, 312-323, 357-362, 391, and 395-402, referring to SEQ ID NO: 3 for numbering, or amino acid residues 1-4, 36, 38, 91-97, 226-228, 251-259, 280-284, 311-322, 356-361, 363, 391, and 395-402, referring to SEQ ID NO: 4 for numbering.
19. Described herein is the variant α-amylase polypeptide of preceding claims 17 or 18, wherein the mutation is in an amino acid residue corresponding to amino acid residues 92, 251, 254, 256, 317, 318, 320, or 321, referring to SEQ ID NO: 4 for numbering.
20. Described herein is the variant α-amylase polypeptide of any of preceding claims 17-19, wherein the mutation is the substitution of the wild-type residue at a position corresponding to R251or K256 with a different amino acid residue, referring to SEQ ID NO: 4 for numbering.
21. Described herein is the variant α-amylase polypeptide of any of preceding paragraphs 17-19, wherein the mutation is the substitution of the wild-type residue at a position corresponding to position 92 in SEQ ID NO: 4 with L, the substitution of the wild-type residue at a position corresponding to position 251 in SEQ ID NO: 4 with A, C, D, E, F, G, H, L, M, N, Q, S, T, or W, the substitution of the wild-type residue at a position corresponding to position 254 in SEQ ID NO: 4 with H, K, W, or Y, the substitution of the wild-type residue at a position corresponding to position 256 in SEQ ID NO: 4 with A, E, T, or V, the substitution of the wild-type residue at a position corresponding to position 317 in SEQ ID NO: 4 with C or R, the substitution of the wild-type residue at a position corresponding to position 318 in SEQ ID NO: 4 with A, F, H, K, Q, or R, the substitution of the wild-type residue at a position corresponding to position 320 in SEQ ID NO: 4 with A, H, M, N, or P, or the substitution of the wild-type residue at a position corresponding to position 321 in SEQ ID NO: 4 with D, G, I, or T.
22. Described herein is the variant α-amylase polypeptide of any of any of preceding paragraphs 17-19, wherein the mutation corresponds to S92L, R251A, R251C, R251D, R251E, R251F, R251G, R251H, R251L, R251M, R251N, R251Q, R251S, R251T, or R251W, T254H, T254K, T254W, or T254Y, K256A, K256E, K256T, or K256V, S317C or S317R, N318A N318F, N318H, N318K, N318Q, or N318R, T320A, T320H, T320M, T320N, or T320P, or K321D, K321G, K321I, or K321T, referring to SEQ ID NO: 4 for numbering.
23. Described herein is the variant α-amylase polypeptide of any of preceding paragraphs 17-22, wherein the variant further comprises wild-type amino acid residues at one or more positions corresponding to N88, A252, A253, N308, A316, S357, T400, R402, and/or D403, referring to SEQ ID NO: 4 for numbering.
24. Described herein is the variant α-amylase polypeptide of any of preceding paragraphs 17-23, wherein the variant further comprises wild-type amino acid residues at one or more positions corresponding to N4, G5, T38, N93, G94, I95, Q96, V97, Y230, G255, V315, P319, A322, L354, T355, R356, G359, Y396, A397, Y398, G399, and/or Q401 in SEQ ID NO: 4, referring to SEQ ID NO: 4 for numbering.
25. Described herein is the variant α-amylase polypeptide of any of preceding paragraphs 17-24, wherein the variant further comprises N at position 88, A at position 252, A at position 253, N at position 308, A at position 316, S at position 357, T at position 400, R at position 402, and/or D at position 403, referring to SEQ ID NO: 4 for numbering.
26. Described herein is the variant α-amylase polypeptide of any of preceding paragraphs 17-25, wherein the variant further comprises N at position 4, G at position 5, T at position 38, N at position 93, G at position 94, I at position 95, Q at position 96, V at position 97, Y at position 230, G at position 255, V at position 315, P at position 319, A at position 322, L at position 354, T at position 355, R at position 356, G at position 359, Y at position 396, A at position 397, Y at position 398, G at position 399, and/or Q at position 401, referring to SEQ ID NO: 4 for numbering.
27. Described herein is the variant of any of preceding paragraphs 17-26, wherein the variant has at least 60%, at least 70%, at least 80%, or at least 90% amino acid sequence identity to SEQ ID NO: 1, 2, 3, 4, or 5.
28. Described herein is the variant of any of preceding paragraphs 17-27, wherein the parent has at least 60%, at least 70%, at least 80%, or at least 90% amino acid sequence identity to SEQ ID NO: 1, 2, 3, 4, or 5.
29. Described herein is the variant of any of preceding paragraphs 17-28, wherein the variant exhibits improved starch liquefaction, starch saccharification, or cleaning performance compared to parent.
30. Described herein is the variant of any of preceding paragraphs 17-29, wherein the variant exhibits increased hydrolysis activity on an amylose substrate compared to the parent.
31. Also described herein is a composition comprising the variant α-amylase polypeptide of any of preceding paragraphs 17-30, and at least one formulation agent.

### BRIEF DESCRIPTION OF THE DRAWINGS

Figure 1 is a ribbon model of the engineered amylase derived from *Pyrococcus woesei,* showing the location of a bound cyclodextrin.
Figure 2 shows the amino acid sequence and the location of secondary structure features in the engineered *P. woesei* amylase.
Figure 3 is an alignment of several Family 13 α-amylases [*Geobacillus* (formerly *Bacillus*) *stearothermophilus* α-amylase, *Bacillus licheniformis* α-amylase (LAT) α-amylase, and *Cytophaga* sp. α-amylase (AAF00561.1, GI# 6006681)] performed using Clustal W with default parameters.
Figure 4 is a stick structure of *B. stearothermophilis* amylase (AmyS) showing the groove residues on the left side of the molecule highlighted as bold sticks. The substrate binding region (*i.e.*, active site cleft) is on the right side of the molecule.
Figure 5 a stick structure of *B. stearothermophilis* amylase (AmyS) looking down at the groove, with the groove forming residues highlighted as bold sticks.
Figure 6 is a histogram showing the distribution of PI for activity values of all SEL variants having a PI for expression >0.3.
Figure 7 is a histogram showing the distribution of PI for activity values of SEL variants having substitutions only in groove positions and having a PI for expression >0.3.
Figures 8A and 8B are a table showing the results of screening individual variants obtained from the SEL library using an amylose substrate.
Figures 9A and 9B are a table showing the results of screening individual variants obtained from the SEL library using an amylopectin substrate.

### BRIEF DESCRIPTION OF THE SEQUENCES

SEQ ID NO: 1 sets forth the amino acid sequence of the mature form of *Pyrococcus woesei* amylase.
SEQ ID NO: 2 sets forth the amino acid sequence of the mature form of *Bacillus licheniformis* α-amylase (LAT) α-amylase.
SEQ ID NO: 3 sets forth the amino acid sequence of the mature form of *Geobacillus* (formerly *Bacillus*) *stearothermophilus* α-amylase.
SEQ ID NO: 4 sets forth the amino acid sequence of the mature form of *Cytophaga* sp. α-amylase (AAF00561.1, GI# 6006681).
SEQ ID NO: 5 sets forth the amino acid sequence of the mature form of a variant P. *woesei* amylase, uPWA.

### DETAILED DESCRIPTION

### 1. Introduction

The present methods relate to variant α-amylase polypeptides comprising mutations in a newly discovered starch-binding groove located on the opposite site of the molecule with respect to the substrate binding site. Mutations in the starch-binding groove alter the binding of the variant α-amylase polypeptides to starch bundles, thereby altering the performance of the molecules in terms of, *e.g*., activity, thermal stability, pH stability, detergent stability, calcium dependence, and the like.

These and other aspects of the methods are described in detail, below.

### 2. Definitions and abbreviations

In accordance with this detailed description, the following abbreviations and definitions apply. Note that the singular forms "a," "an," and "the" include plural referents unless the context clearly dictates otherwise. Thus, for example, reference to "an enzyme" includes a plurality of such enzymes, and reference to "the dosage" includes reference to one or more dosages and equivalents thereof known to those skilled in the art, and so forth.

The present document is organized into a number of sections for ease of reading; however, the reader will appreciate that statements made in one section may apply to other sections. In this manner, the headings used for different sections of the disclosure should not be construed as limiting.

Unless defined otherwise, all technical and scientific terms used herein have the same meaning as commonly understood by one of ordinary skill in the art. The following abbreviations and/or terms are defined for clarity:

### 2.1 Abbreviations/acronyms

The following abbreviations/acronyms have the following meanings unless otherwise specified:
- cDNA: complementary DNA
- DNA: deoxyribonucleic acid
- EC: enzyme commission
- EDTA: ethylenediaminetetraacetic acid
- GA: glucoamylase
- IPTG: isopropyl β-D-thiogalactoside
- kDa: kiloDalton
- LAT: *B. licheniformis* amylase
- MW: molecular weight
- MWU: modified Wohlgemuth unit; 1.6x10⁻⁵ mg/MWU = unit of activity
- NOBS: nonanoyloxybenzenesulfonate
- NTA: nitriloacetic acid
- PEG: polyethyleneglycol
- pI: isoelectric point
- PVA: poly(vinyl alcohol)
- PVP: poly(vinylpyrrolidone)
- RNA: ribonucleic acid
- SAS: alkanesulfonate
- SDS-PAGE: sodium dodecyl sulfate polyacrylamide gel electrophoresis
- sp.: species
- w/v: weight/volume
- w/w: weight/weight
- v/v: volume/volume
- wt%: weight percent
- °C: degrees Centigrade
- H₂O: water
- dH₂O or DI: deionized water
- dIH₂O: deionized water, Milli-Q filtration
- g or gm: grams
- µg: micrograms
- mg: milligrams
- kg: kilograms
- µL and µl: microliters
- mL and ml: milliliters
- mm: millimeters
- Å: Angstrom
- µm: micrometer
- M: molar
- mM: millimolar
- µM: micromolar
- U: units
- sec: seconds
- min(s): minute/minutes
- hr(s): hour/hours
- Ncm: Newton centimeter
- ETOH: ethanol
- eq.: equivalents
- N: normal
- ds or DS: dry solids content
- uPWA: variant α-amylase derived from *Pyrococcus woesei*
- PWA: α-amylase from *Pyrococcus woesei*
- MWCO: molecular weight cut-off
- SSRL: Stanford Synchrotron Radiation Lightsource
- PDB: Protein Database
- CAZy: Carbohydrate-Active Enzymes database
- Tris-HCl: tris(hydroxymethyl)aminomethane hydrochloride
- HEPES: 4-(2-hydroxyethyl)-1-piperazineethanesulfonic acid

### 2.2 Definitions

The terms "amylase" or "amylolytic enzyme" refer to an enzyme that is, among other things, capable of catalyzing the degradation of starch. α-amylases are hydrolases that cleave the α-D-(1→4) O-glycosidic linkages in starch. Generally, α-amylases (EC 3.2.1.1; α-D-(1→4)-glucan glucanohydrolase) are defined as endo-acting enzymes cleaving α-D-(1→4) O-glycosidic linkages within the starch molecule in a random fashion yielding polysaccharides containing three or more (1-4)-α-linked D-glucose units. In contrast, the exo-acting amylolytic enzymes, such as β-amylases (EC 3.2.1.2; α-D-(1→4)-glucan maltohydrolase) and some product-specific amylases like maltogenic α-amylase (EC 3.2.1.133) cleave the polysaccharide molecule from the non-reducing end of the substrate. β-amylases, α-glucosidases (EC 3.2.1.20; α-D-glucoside glucohydrolase), glucoamylase (EC 3.2.1.3; α-D-(1→4)-glucan glucohydrolase), and product-specific amylases like the maltotetraosidases (EC 3.2.1.60) and the maltohexaosidases (EC 3.2.1.98) can produce malto-oligosaccharides of a specific length. Some bacterial α-amylases predominantly produce maltotetraose (G4), maltopentaose (G5) or maltohexaose (G6) from starch and related α-1,4-glucans, while most α-amylases further convert them to glucose and or maltose as final products. For the purposes of the present compositions and methods, no distinction is made between true, endo-acting, α-amylases and G4, G5, G6, and similar amylases.

The expression "Family 13-like amylase" refers to any amylase classified as a CAZy Family 13 amylase, having at least 60% amino acid sequence identity to SEQ ID NOs: 1-5, and/or heretofore described as "Termamyl-like." The expression "Family 13 amylase" refers to any amylase classified as CAZy Family 13 amylase.

The expression "amylase having the fold of *Bacillus* sp. amylase" refers to any amylase having a structure that is substantially super-imposable on SEQ ID NOs: 2 or 3.

The expression "Termamyl-like" is as used in various published patent applications and patents.

As used herein the term "starch" refers to any material comprised of the complex polysaccharide carbohydrates of plants, comprised of amylose and amylopectin with the formula (C₆H₁₀O₅)ₓ, wherein X can be any number. The term includes plant-based materials such as grains, grasses, tubers and roots, and more specifically materials obtained from wheat, barley, corn, rye, rice, sorghum, brans, cassava, millet, potato, sweet potato, and tapioca.

The term "starch bundle" refers to a form of starch in which individual starch polymers are arranged, *e.g.,* by gelatinization followed by retrogradation, such that they are resistant to enzymatic hydrolysis by α-amylase enzymes. In some cases, the polymers in starch bundles are parallel *(i.e.,* aligned), precluding access by enzymes.

The term "amylose" refers to an unbranched starch substrate consisting of glucose residues in α-1,4 linkages.

The term "amylopectin" refers to a branched starch substrate consisting of α-1,6 linkages and α-1,4 linkages.

The terms, "wild-type," "parental," or "reference," with respect to a polypeptide, refer to a naturally-occurring polypeptide that does not include a man-made substitution, insertion, or deletion at one or more amino acid positions. Similarly, the terms "wild-type," "parental," or "reference," with respect to a polynucleotide, refer to a naturally-occurring polynucleotide that does not include a man-made nucleoside change. However, note that a polynucleotide encoding a wild-type, parental, or reference polypeptide is not limited to a naturally-occurring polynucleotide, and encompasses any polynucleotide encoding the wild-type, parental, or reference polypeptide. Substitutions, insertions, or deletions may be made by "shuffling."

The expression "one or several" means less than 10.

The term "variant," with respect to a polypeptide, refers to a polypeptide that differs from a specified wild-type, parental, or reference polypeptide in that it includes a man-made substitution, insertion, or deletion at one or more amino acid positions. Similarly, the term "variant," with respect to a polynucleotide, refers to a polynucleotide that differs in nucleotide sequence from a specified wild-type, parental, or reference polynucleotide. The identity of the wild-type, parental, or reference polypeptide or polynucleotide will be apparent from context. Variants may be made by "shuffling."

The term "recombinant," when used in reference to a subject cell, nucleic acid, protein or vector, indicates that the subject has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell, or express native genes at different levels or under different conditions than found in nature.

The terms "recovered," "isolated," and "separated," refer to a compound, protein (polypeptides), cell, nucleic acid, amino acid, or other specified material or component that is removed from at least one other material or component with which it is naturally associated as found in nature.

As used herein, the term "purified" refers to material (*e.g*., an isolated polypeptide or polynucleotide) that is in a relatively pure state, *e.g.,* at least about 90% pure, at least about 95% pure, at least about 98% pure, or even at least about 99% pure.

The term "enhanced stability" or "increased stability" in the context of an oxidizing environment, the presence of chelators, the presence of detergents, exposure to elevated temperatures, and/or exposure to pH extremes, means that a subject amylase retains more amylolytic activity over time compared to another *(i.e.,* reference) amylase.

The terms "thermostable" and "thermostability," with reference to an enzyme, refer to the ability of the enzyme to retain activity after exposure to an elevated temperature. The thermostability of an enzyme, such as an amylase enzyme, is measured by its half-life (t_{1/2}) given in minutes, hours, or days, during which half the enzyme activity is lost under defined conditions. The half-life may be calculated by measuring residual amylase activity following exposure to *(i.e.,* challenge by) an elevated temperature.

A "pH range," with reference to an enzyme, refers to the range of pH values under which the enzyme exhibits catalytic activity.

As used herein, the terms "pH stable" and "pH stability," with reference to an enzyme, relate to the ability of the enzyme to retain activity over a wide range of pH values for a predetermined period of time *(e.g.,* 15 min., 30 min., 1 hour).

As used herein, the term "amino acid sequence" is synonymous with the terms "polypeptide," "protein," and "peptide," and are used interchangeably. Where such amino acid sequences exhibit activity, they may be referred to as an "enzyme." The conventional one-letter or three-letter codes for amino acid residues are used, with amino acid sequences being presented in the standard amino-to-carboxy terminal orientation *(i.e.,* N→C).

The term "nucleic acid" encompasses DNA, RNA, heteroduplexes, and synthetic molecules capable of encoding a polypeptide. Nucleic acids may be single stranded or double stranded, and may be chemical modifications. The terms "nucleic acid" and "polynucleotide" are used interchangeably. Because the genetic code is degenerate, more than one codon may be used to encode a particular amino acid, and the present compositions and methods encompass nucleotide sequences that encode a particular amino acid sequence. Unless otherwise indicated, nucleic acid sequences are presented in 5'-to-3' orientation.

The term "homologue" refers to an entity having a specified degree of identity with the subject amino acid sequences and the subject nucleotide sequences. A homologous sequence is taken to include an amino acid sequence that is at least 60%, at least 65%, at least 70%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98% or even at least 99% identical to the subject sequence, using Clustal W (Thompson J.D. et al. (1994) Nucleic Acids Res. 22:4673-4680) with default parameters, *i.e.:*

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF |

The term "hybridization" refers to the process by which one strand of nucleic acid base pairs with a complementary strand, as occurs during blot hybridization techniques and PCR techniques. Stringent hybridization conditions are exemplified by the following: 65°C and 0.1X SSC (where IX SSC = 0.15 M NaCl, 0.015 M Na₃ citrate, pH 7.0).

The term "saccharification" refers to enzymatic conversion of starch to glucose.

The term "liquefaction" refers to the stage in starch conversion in which gelatinized starch is hydrolyzed to give low molecular weight soluble dextrins. The term "degree of polymerization" (DP) refers to the number (n) of anhydroglucopyranose units in a given saccharide. Examples of DP1 are the monosaccharides glucose and fructose. Examples of DP2 are the disaccharides maltose and sucrose. For purposes herein "comparable liquefaction processes" are those conducted under comparable conditions, preferably conditions standardized for temperature, pH, substrate concentration, calcium ion concentration and the like. Preferably comparable liquefaction processes are compared on an equal protein or equal activity units basis, however, is some embodiments, either protein, or activity units, or both may vary in comparable liquefaction processes. The skilled artisan will understand the bases on which liquefaction processes may be "comparable".

During liquefaction processes, the viscosity of the starch slurry is frequently used as a measure of the conversion of the starch into smaller DP units. Sometimes herein the expression "initial viscosity" is used. The skilled artisan will appreciate that this is a term of art, and what is intended is not literally the initial viscosity, but rather the peak viscosity which occurs for example at around the point of gelatinization of the substrate. Initial viscosity is used to distinguish from the "final viscosity" which is the viscosity of a substrate, *e.g*., starch slurry, at the conclusion of a liquefaction process. Thus, as is apparent from looking at a graph of the viscosity over the time-course of a liquefaction process, the initial or peak viscosity occurs not immediately, but after a certain passage of time, *e.g.,* less than 50% of the overall time, and more preferably within about the first 1/3 or even 1/4 or 1/5 of the total time of liquefaction. For example, in a liquefaction exemplified herein, the peak viscosity typically occurs within the first ten minutes, after which time the enzyme is added and the viscosity begins to drop. As the starch granules open up during the gelatinization process, the interior of the granules become more accessible to the amylase activity and more cleavage, resulting in a drop in viscosity.

The term "dry solids content" (ds) refers to the total amount of solids in a slurry, on a dry weight basis. Dry solids content and dry weight basis are usually expressed as the weight of the subject material as a percentage of the weight of the total dry material. The term "slurry" refers to a mixture containing insoluble solids in a liquid, typically water or a similar solvent. Starch or flour is frequently suspended in a water-based solution to form a slurry for testing amylases, or for liquefaction processes.

The term "dextrose equivalent" or "DE" is defined as the percentage of reducing sugar as a fraction of total carbohydrate. The term "degree of polymerization" or "DP" refers to the size of products of amylase degradation of starch. The higher the DP, the more complex the carbohydrate.

The term "blend" or "enzyme blend" refers to a composition comprising a mixture of two or more enzymes providing therefore a combined catalytic activity that entails the activity of each of the enzymes present in the mixture. Enzyme blends need not have equal amounts of each of the two or more enzymes, but enzyme blends may be formulated on an equal protein, or equal activity basis, if desired. The combined catalytic activity may be merely additive or averaged, or may be antagonistic or synergistic. Preferred blends for use herein provide at least additive catalytic activity, and more preferably, synergistic catalytic effects.

For purposes herein, a "comparable liquefaction process" means a similar processes performed under controlled and specified conditions, (*e.g*., with respect to temperature, pH, calcium ion concentration, and substrate concentration) using a different or "control" amylase, and which can be compared to a subject liquefaction process.

As used herein, one "alpha amylase unit (AAU)" is the amount of bacterial alpha amylase activity required to hydrolyze 10 mg of starch per minute under specified conditions.

As used herein, the terms "transformed," "stably transformed," and "transgenic," used with reference to a cell means that the cell contains a non-native (*e.g*., heterologous) nucleic acid sequence integrated into its genome or carried as an episome that is maintained through multiple generations.

The term "introduced" in the context of inserting a nucleic acid sequence into a cell, means "transfection", "transformation" or "transduction," as known in the art.

A "host strain" or "host cell" is an organism into which an expression vector, phage, virus, or other DNA construct, including a polynucleotide encoding a polypeptide of interest *(e.g.,* an amylase) has been introduced. Exemplary host strains are bacterial cells. The term "host cell" includes protoplasts created from cells, such as those of a *Bacillus* sp.

The term "heterologous" with reference to a polynucleotide or protein refers to a polynucleotide or protein that does not naturally occur in a host cell.

The term "endogenous" with reference to a polynucleotide or protein refers to a polynucleotide or protein that occurs naturally in the host cell.

As used herein, the term "expression" refers to the process by which a polypeptide is produced based on a nucleic acid sequence. The process includes both transcription and translation.

A "selective marker" or "selectable marker" refers to a gene capable of being expressed in a host to facilitate selection of host cells carrying the gene. Examples of selectable markers include but are not limited to antimicrobials (*e.g*., hygromycin, bleomycin, or chloramphenicol) and/or genes that confer a metabolic advantage, such as a nutritional advantage on the host cell.

A "vector" refers to a polynucleotide sequence designed to introduce nucleic acids into one or more cell types. Vectors include cloning vectors, expression vectors, shuttle vectors, plasmids, phage particles, cassettes and the like.

An "expression vector" refers to a DNA construct comprising a DNA sequence encoding a polypeptide of interest, which coding sequence is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences that control termination of transcription and translation.

The term "operably linked" means that specified components are in a relationship (including but not limited to juxtaposition) permitting them to function in an intended manner. For example, a regulatory sequence is operably linked to a coding sequence if the expression of the coding sequence is under control of the regulatory sequences.

A "signal sequence" is a sequence of amino acids attached to the N-terminal portion of a protein, which facilitates the secretion of the protein outside the cell. The mature form of an extracellular protein lacks the signal sequence, which is cleaved off during the secretion process.

As used herein, "biologically active" refer to a sequence having a specified biological activity, such an enzymatic activity.

As used herein, "a cultured cell material comprising an α-amylase polypeptide," or similar language, refers to a cell lysate or supernatant (including media) that includes an α-amylase polypeptide as a component. The cell material is preferably from a heterologous host that is grown in culture for the purpose of producing the α-amylase polypeptide.

### 3. Identification of a new starch-binding site in an α-amylase

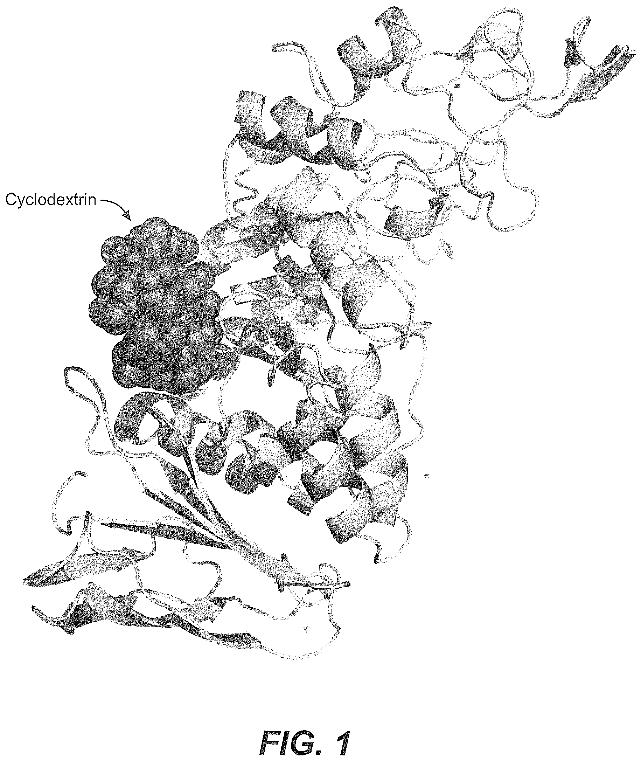

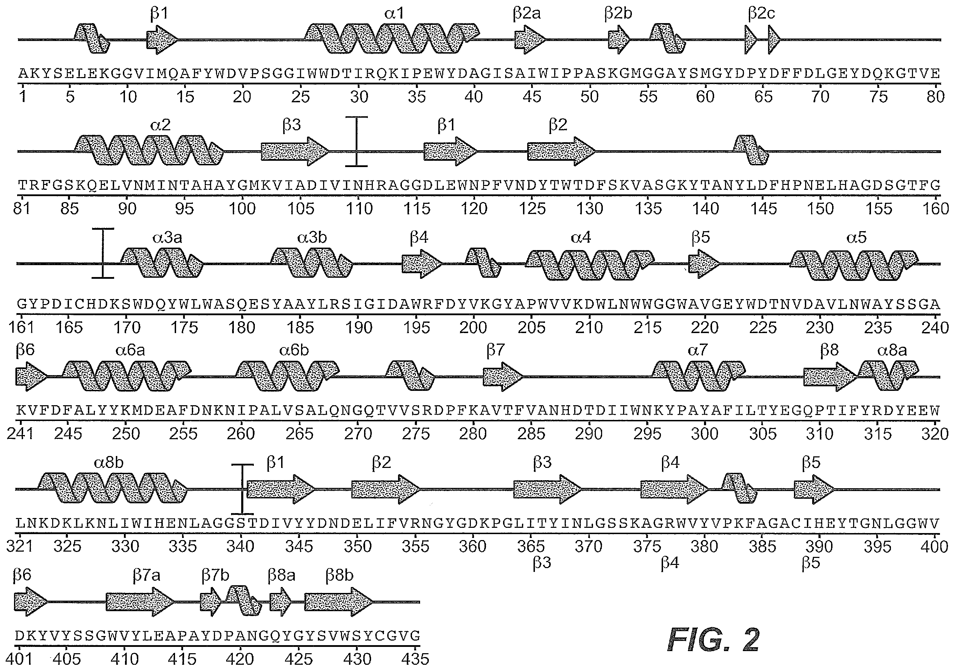

Referring to the appended Examples, the three-dimensional structure of an engineered variant α-amylase of the hyperthermophilic archaeon, *Pyrococcus woesei,* was determined at 2 Å resolution (Figures 1 and 2). Unexpectedly, a cyclodextrin molecule was found associated with the enzyme in the crystals (Figure 1). Cyclodexrin was not part of the crystallization medium, suggesting that the cyclodextrin bound to the molecule during expression or purification, and remained with the molecule during crystallization. The cyclodextrin molecule was not associated with the substrate binding site. Instead, it was bound in a groove located on the opposite side of the molecule from the substrate-binding site.

Notably, several mutations in the variant *P. woesei* α-amylase *(i.e.,* D358Y, S359G, R360D, R361K, and P372S) are located close to the bound cyclodextrin, suggesting that mutations at these positions modulate binding. Adjacent residues within 5 Å of the one or more atoms of the bound cyclodextrin that would be expected to modulate this binding include Gln7, Lys8, Gly9, Lys241, Pro278, Phe279, Glu307, Gly308, Gln309, Gly338, Gly339, Ser340, Arg355 and Tyr358. Additional residues that form the starch-binding groove include residues Ser4, Glu5, Tyr236, Gln268, Ser275, Arg276, Asp277, Lys280, Tyr306, Glu334, Thr341, Asp342, Ile343, Asn 356, Asp360, and Lys361.

### 4. Variant α-amylases with mutations in the starch-binding groove

Without being limited to a theory, the present compositions and methods are based on the hypothesis that in the three-dimensional structure described, above, cyclodextrin mimics a starch helical bundle, to which it is advantageous for the α-amylase to bind. The presence of the starch-binding region allows the amylase to associate with a starch bundle, and perhaps even slide along the starch bundle, to disrupted locations where the enzyme can hydrolyze starch chains, which bind at the canonical substrate binding site. The ability to slide to regions favoring hydrolysis would reduce the diffusional freedom from three-dimensional diffusion in solution to one-dimensional diffusion along starch molecules. This would enhance the processivity of the molecule to hydrolyze starch as would be evidenced by improved starch hydrolysis. Starch-binding via the groove may even promote disruption of a starch bundle, thereby increasing hydrolysis. Furthermore, starch-binding via the groove may confer stability to the α-amylase, which may be evidenced by, *e.g*., increased thermal stability, increased pH stability, increased detergent stability, decreased calcium dependence, and the like.

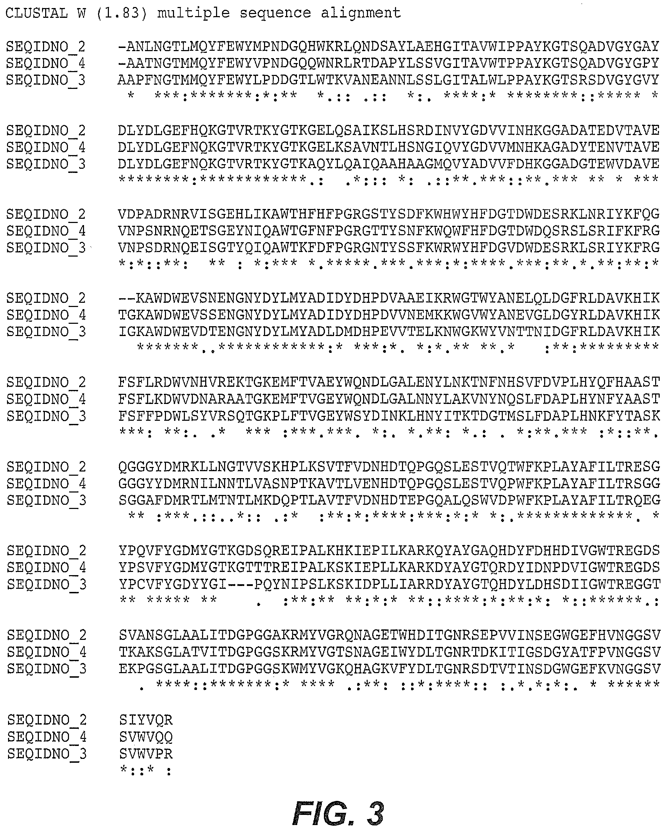

The three-dimensional structures of Carbohydrate-Active Enzymes database (CAZy) Family 13 amylases are highly conserved, consisting of three domains (see, *e.g*., Henrissat, B. (1991) Biochem. J. 280:309-316; Henrissat, B. and Bairoch, A. (1996) Biochem. J. 316:695-696; Henrissat, B. and Davies, G. (1997) Curr. Opin. Biotech. 7:637-644; Nagano, N. et al. (2001) Protein Eng. 14:845-855; Pujadas, G. and Palau, J. (2001) Mol. Biol. Evol. 18:38-54; WO 94/02597). Referring to Figure 2 the A Domain is a TIM barrel structure at the N-terminus of the polypeptide. The B Domain is a large loop region between the third β-strand (β_{A3}) and the third α-helix (α_{A3}) in the A Domain. The C Domain includes the C-terminus of the polypeptide. The amylases from *Bacillus licheniformis* (AmyL), *Bacillus stearothermophilus* (AmyS), and *Cytophaga* sp. are both member of this Family. An alignment of these amylases is shown in Figure 3. The structures of these amylases are exemplified by entries 1hxv and 1bli, respectively, in the Protein Data Bank, which show even more pronounced groove features located in the corresponding regions on these molecules.

In each of these enzymes, there are about 44 homologous residues forming the starch-binding groove. These residues are in the short α-helix preceding the first β-strand in the A domain, the loop between the sixth α-helix and the seventh β-strand in the A domain, the loop between the seventh α-helix and the eighth β-strand in the A domain, and the loop connecting the A domain and the C domain. In a variant *P. woesei* amylase, these residues correspond to residues Gln7, Lys8, Gly9, Lys241, Pro278, Phe279, Glu307, Gly308, Gln309, Gly338, Gly339, Ser340, Arg355 and Tyr358. Additional residues that form the starch-binding groove include residues Ser4, Glu5, Tyr236, Gln268, Ser275, Arg276, Asp277, Lys280, Tyr306, Glu334, Thr341, Asp342, Ile343, Asn 356, Asp360, and Lys361 (referring to SEQ ID NO: 5 for numbering). In *B. licheniformis* amylase, these residues correspond to residues 1-6, 36, 38, 91-97, 224-226, 249-257, 278-282, 309- 320, 354-359, 391, and 395-402 (referring to SEQ ID NO: 2 for numbering). In *B. stearothermophilus* amylase, these residues correspond to residues 1-6, 37, 39, 92-98, 227-229, 252-260, 281-285, 312-323, 357-362, 391, and 395-402(referring to SEQ ID NO: 3 for numbering). In *Cytophaga sp.* amylase these residues correspond to residues 1-4, 36, 38, 91-97, 226-228, 251-259, 280-284, 311-322, 356-361, 363, 391, and 395-402 (referring to SEQ ID NO: 4).
The present variants include substitutions of the wild-type residue at a position corresponding to position 92 in SEQ ID NO: 4 with L, the substitution of the wild-type residue at a position corresponding to position 251 in SEQ ID NO: 4 with A, C, D, E, F, G, H, L, M, N, Q, S, T, or W, the substitution of the wild-type residue at a position corresponding to position 254 in SEQ ID NO: 4 with H, K, W, or Y, the substitution of the wild-type residue at a position corresponding to position 256 in SEQ ID NO: 4 with A, E, T, or V, the substitution of the wild-type residue at a position corresponding to position 317 in SEQ ID NO: 4 with C or R, the substitution of the wild-type residue at a position corresponding to position 318 in SEQ ID NO: 4 with A, F, H, K, Q, or R, the substitution of the wild-type residue at a position corresponding to position 320 in SEQ ID NO: 4 with A, H, M, N, or P, and/or the substitution of the wild-type residue at a position corresponding to position 321 in SEQ ID NO: 4 with D, G, I, or T.

In some embodiments, the present variants do not include substitutions of the wild-type amino acid residues at one or more positions corresponding to N88, A252, A253, N308, A316, S357, T400, R402, and D403 in SEQ ID NO: 4. In some embodiments, the present variants do not include substitutions of the wild-type amino acid residues at one or more positions corresponding to N4, G5, T38, N93, G94, I95, Q96, V97, Y230, G255, V315, P319, A322, L354, T355, R356, G359, Y396, A397, Y398, G399, and Q401 in SEQ ID NO: 4.

In some embodiments, the present variants include one or more of the following residues at the following positions corresponding to SEQ ID NO: 4: N at position 88, A at position 252, A at position 253, N at position 308, A at position 316, S at position 357, T at position 400, R at position 402, and D at position 403 in SEQ ID NO: 4. In some embodiments, the present variants include all of the aforementioned residues at the indicated positions. In some embodiments, the present variants include one or more of the following residues at the following positions corresponding to SEQ ID NO: 4: N at position 4, G at position 5, T at position 38, N at position 93, G at position 94, I at position 95, Q at position 96, V at position 97, Y at position 230, G at position 255, V at position 315, P at position 319, A at position 322, L at position 354, T at position 355, R at position 356, G at position 359, Y at position 396, A at position 397, Y at position 398, G at position 399, and Q at position 401 in SEQ ID NO: 4. In some embodiments, the present variants include all of the aforementioned residues at the indicated positions.

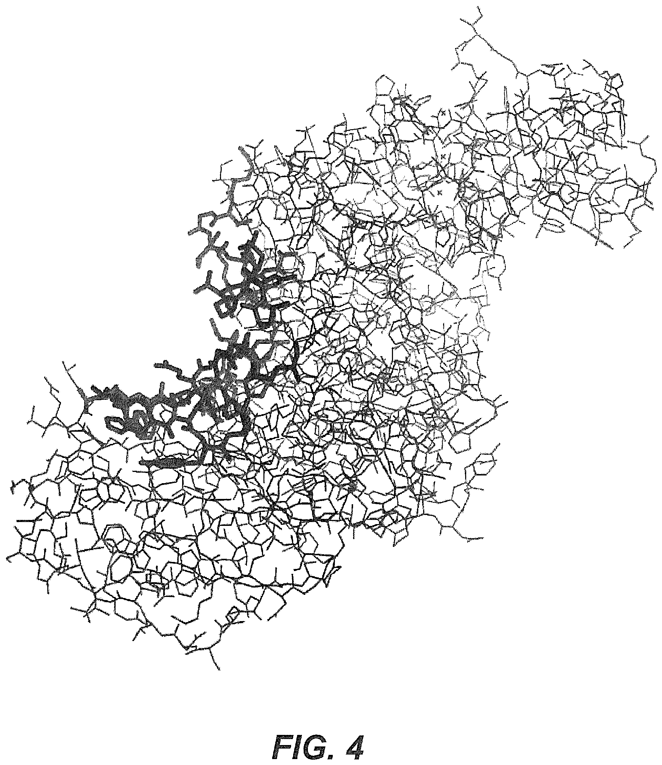

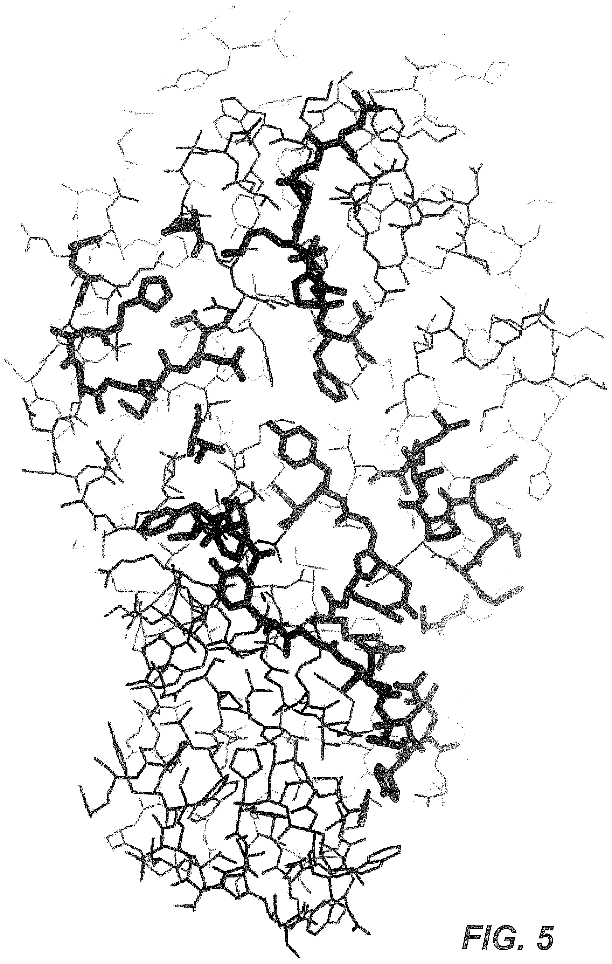

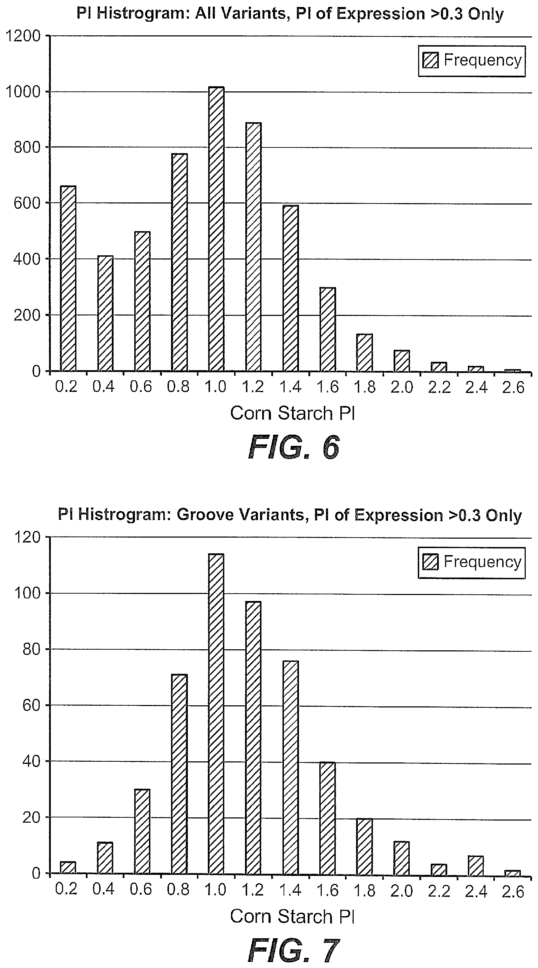

Corresponding positions in other amylases can be determined by amino acid sequence alignment. Figures 4 and 5 illustrate the location of the groove in *B. stearothermophilus* amylase. The homologous residues in other amylases can be determined by structural alignment, or by primary structure alignment, as illustrated by Figure 6.
Variant *P. woesei* amylase, uPWA (SEQ ID NO: 5) 
AKYSELEKGG VIMQAFYWDV PSGGIWWDTI RQKIPEWYDA GISAIWIPPA
SKGMGGAYSM GYDPYDFFDL GEYDQKGTVE TRFGSKQELV NMINTAHAYG
MKVIADIVIN HRAGGDLEWN PFVNDYTWTD FSKVASGKYT ANYLDFHPNE
LHAGDSGTFG GYPDICHDKS WDQYWLWASQ ESYAAYLRSI GIDAWRFDYV
KGYAPWVVKD WLNWWGGWAV GEYWDTNVDA VLNWAYSSGA KVFDFALYYK
MDEAFDNKNI PALVSALQNG QTVVSRDPFK AVTFVANHDT DIIWNKYPAY
AFILTYEGQP TIFYRDYEEW LNKDKLKNLI WIHENLAGGS TDIVYYDNDE
LIFVRNGYGD KPGLITYINL GSSKAGRWVY VPKFAGACIH EYTGNLGGWV
DKYVYSSGWV YLEAPAYDPA NGQYGYSVWS YCGVG
*Bacillus licheniformis* α-amylase (LAT; SEQ ID NO: 2): 
ANLNGTLMQY FEWYMPNDGQ HWKRLQNDSA YLAEHGITAV WIPPAYKGTS
QADVGYGAYD LYDLGEFHQK GTVRTKYGTK GELQSAIKSL HSRDINVYGD
VVINHKGGAD ATEDVTAVEV DPADRNRVIS GEHLIKAWTH FHFPGRGSTY
SDFKWHWYHF DGTDWDESRK LNRIYKFQGK AWDWEVSNEN GNYDYLMYAD
IDYDHPDVAA EIKRWGTWYA NELQLDGFRL DAVKHIKFSF LRDWVNHVRE
KTGKEMFTVA EYWQNDLGAL ENYLNKTNFN HSVFDVPLHY QFHAASTQGG
GYDMRKLLNG TVVSKHPLKS VTFVDNHDTQ PGQSLESTVQ TWFKPLAYAF
ILTRESGYPQ VFYGDMYGTK GDSQREIPAL KHKIEPILKA RKQYAYGAQH

DYFDHHDIVG WTREGDSSVA NSGLAALITD GPGGAKRMYV GRQNAGETWH
DITGNRSEPV VINSEGWGEF HVNGGSVSIY VQR
*Geobacillus* (formerly *Bacillus*) *stearothermophilus* amylase (SEQ ID NO: 3): 
AAPFNGTMMQ YFEWYLPDDG TLWTKVANEA NNLSSLGITA LWLPPAYKGT
SRSDVGYGVY DLYDLGEFNQ KGTVRTKYGT KAQYLQAIQA AHAAGMQVYA
DVVFDHKGGA DGTEWVDAVE VNPSDRNQEI SGTYQIQAWT KFDFPGRGNT
YSSFKWRWYH FDGVDWDESR KLSRIYKFRG IGKAWDWEVD TENGNYDYLM
YADLDMDHPE VVTELKNWGK WYVNTTNIDG FRLDAVKHIK FSFFPDWLSY
VRSQTGKPLF TVGEYWSYDI NKLHNYITKT DGTMSLFDAP LHNKFYTASK
SGGAFDMRTL MTNTLMKDQP TLAVTFVDNH DTEPGQALQS WVDPWFKPLA
YAFILTRQEG YPCVFYGDYY GIPQYNIPSL KSKIDPLLIA RRDYAYGTQH
DYLDHSDIIG WTREGGTEKP GSGLAALITD GPGGSKWMYV GKQHAGKVFY
DLTGNRSDTV TINSDGWGEF KVNGGSVSVW VPR
*Cytophaga* sp. α-amylase (AAF00567.1, GI# 6006681; SEQ ID NO: 4):

AATNGTMMQY FEWYVPNDGQ QWNRLRTDAP YLSSVGITAV WTPPAYKGTS
QADVGYGPYD LYDLGEFNQK GTVRTKYGTK GELKSAVNTL HSNGIQVYGD
VVMNHKAGAD YTENVTAVEV NPSNRNQETS GEYNIQAWTG FNFPGRGTTY
SNFKWQWFHF DGTDWDQSRS LSRIFKFRGT GKAWDWEVSS ENGNYDYLMY
ADIDYDHPDV VNEMKKWGVW YANEVGLDGY RLDAVKHIKF SFLKDWVDNA
RAATGKEMFT VGEYWQNDLG ALNNYLAKVN YNQSLFDAPL HYNFYAASTG
GGYYDMRNIL NNTLVASNPT KAVTLVENHD TQPGQSLEST VQPWFKPLAY
AFILTRSGGY PSVFYGDMYG TKGTTTREIP ALKSKIEPLL KARKDYAYGT
QRDYIDNPDV IGWTREGDST KAKSGLATVI TDGPGGSKRM YVGTSNAGEI
WYDLTGNRTD KITIGSDGYA TFPVNGGSVS VWVQQ

According to the present disclosure, one or more of the residues in the aforementioned secondary structure motifs, or one or more of the aforementioned amino acid residues, or corresponding residues in another Family 13-like α-amylase, are mutated with the effect of altering the interaction between the starch-binding groove and starch bundles. In some embodiments, a single residue is mutated. In other embodiments, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, or even 44 residues are mutated. In some embodiments, one or more of the residues in the aforementioned secondary structure motifs, or one or more of the aforementioned amino acid residues, or corresponding residues in another Family 13-like α-amylase, is expressly not mutated, while one or more residues are mutated. In some embodiments, one or several of the residues in the aforementioned secondary structure motifs, or one or several of the aforementioned amino acid residues, or corresponding residues in another Family 13-like α-amylase, is expressly not mutated, while one or several residues are mutated.

In some embodiments, the mutations increase the binding of a Family 13-like α-amylase to starch bundles, *e.g.,* by increasing molecular interactions between residues in the starch-binding groove with starch bundles. Such mutations are expected to restrict amylase diffusion and improve processivity, although they may also increase thermal stability, pH stability, or detergent stability, as well as decrease calcium dependence.

In some embodiments, the present amylase is CAZy Family 13 amylase. In some embodiments, the present amylase has the fold of *Bacillus* sp. amylase.

In some embodiments, the present amylase is a variant of a *Cytophaga* sp. amylase having a defined degree of amino acid sequence homology/identity to SEQ ID NO: 4, for example, at least 60%, at least 65%, at least 70%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98% or even at least 99% amino acid sequence homology/identity.

Furthermore, the present amylases may include any number of conservative amino acid substitutions, at positions not specifically mutated. Exemplary conservative amino acid substitutions are listed in the Table 1

**Table 1. Conservative amino acid substitutions**

| *For Amino Acid* | *Code* | *Replace with any of* |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Orn, D-Orn |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | c | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, b-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4- carboxylic acid, D-or L-1-oxazolidine-4-carboxylic acid |
| Serine | s | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

The present amylases may be "precursor," "immature," or "full-length," in which case they include a signal sequence, or "mature," in which case they lack a signal sequence. Mature forms of the polypeptides are generally the most useful. Unless otherwise noted, the amino acid residue numbering used herein refers to the mature forms of the respective amylase polypeptides. The present amylase polypeptides may also be truncated to remove the N or C-termini, so long as the resulting polypeptides retain amylase activity.

The present amylases may be "chimeric" or "hybrid" polypeptides, in that they include at least a portion of a first amylase polypeptide, and at least a portion of a second amylase polypeptide (such chimeric amylases have recently been "rediscovered" as domain-swap amylases). The present amylases may further include heterologous signal sequence, an epitope to allow tracking or purification, or the like. Exemplary heterologous signal sequences are from *B. licheniformis* amylase (LAT), *B. subtilis* (AmyE or AprE), and *Streptomyces* CelA.

Also described herein are nucleic acids encoding any of the described amylase polypeptides. The nucleic acid may encode a particular amylase polypeptide, or an amylase having a specified degree of amino acid sequence identity to the particular amylase. In one example, the nucleic acid encodes an amylase having at least 60%, at least 65%, at least 70%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98% or even at least 99% homology/identity to a reference amylase. It will be appreciated that due to the degeneracy of the genetic code, a plurality of nucleic acids may encode the same polypeptide.

The nucleic acid may also have a specified degree of homology to an exemplary polynucleotide encoding an α-amylase polypeptide. For example, the nucleic acid may have at least 60%, at least 65%, at least 70%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98% or even at least 99% nucleotide sequence identity to the exemplary sequence. In another example, the nucleic acid hybridizes under stringent or very stringent conditions to the exemplary sequence. Such conditions are described here but are also well known in the art.

Nucleic acids may encode a "full-length" ("fl" or "FL") amylase, which includes a signal sequence, only the mature form of an amylase, which lacks the signal sequence, or a truncated form of an amylase, which lacks the N or C-terminus of the mature form.

A nucleic acid that encodes an α-amylase can be operably linked to various promoters and regulators in a vector suitable for expressing the α-amylase in host cells. Exemplary promoters are from *B. licheniformis* amylase (LAT), *B. subtilis* (AmyE or AprE), and *Streptomyces* CelA. Such a nucleic acid can also be linked to other coding sequences, *e.g.,* to encode a chimeric polypeptide.

### 5. Methods for making variant amylases

An aspect of the present disclosure is a method for making a variant amylase by specifically mutating one or more residues in the starch bundle-binding groove, which are identified above. In this manner, mutations are introduced to a common structural feature in the amylase molecule to bring about a desired effect relating to starch-binding. Mutations can be made using well-known molecular biology techniques and the resulting variant amylases can be expressed as secreted polypeptides using standard methods. For example, methods of producing and purifying proteins that are secreted in to the culture medium from *Bacillus* are known in the art, as are suitable host cells for producing amylases. Exemplary methods for producing the amylases are disclosed below.

### 5.1. Materials and Methods for Producing Amylases

A polypeptide can be expressed using an expression vector which will typically include control sequences including a suitable promoter, operator, ribosome binding site, translation initiation signal, and, optionally, a repressor gene or various activator genes. A large number of vectors are commercially available for use with recombinant DNA procedures, and the choice of vector will often depend on the host cell into which it is to be introduced. The vector may be an autonomously replicating vector, *i.e.*, a vector that exists as an extrachromosomal entity, the replication of which is independent of chromosomal replication, *e.g.*, a plasmid, a bacteriophage or an extrachromosomal element, mini-chromosome or an artificial chromosome. Alternatively, the vector may be one which, when introduced into an isolated host cell, is integrated into the host cell genome and replicated together with the chromosome(s) into which it has been integrated. The integrated gene may also be amplified to create multiple copies of the gene in the chromosome by use of an amplifiable construct driven by antibiotic selection or other selective pressure, such as an essential regulatory gene or by complementation through dose effect of an essential metabolic pathway gene.

In the vector, the DNA sequence should be operably linked to a suitable promoter sequence. The promoter may be any DNA sequence that shows transcriptional activity in the host cell of choice and may be derived from genes encoding proteins either homologous or heterologous to the host cell. Exemplary promoters for directing the transcription of the DNA sequence encoding an amylase, especially in a bacterial host, are the promoter of the lac operon of *E. coli,* the *Streptomyces coelicolor* agarase gene dagA or celA promoters, the promoters of the *Bacillus licheniformis* α-amylase gene (amyL), the promoters of the *Bacillus stearothermophilus* maltogenic amylase gene (amyM), the promoters of the *Bacillus amyloliquefaciens* α-amylase (amyQ), the promoters of the *Bacillus subtilis* xylA and xylB genes *etc.* For transcription in a fungal host, examples of useful promoters are those derived from the gene encoding *Aspergillus oryzae* TAKA amylase, *Rhizomucor miehei* aspartic proteinase, *Aspergillus niger* neutral α-amylase, A. *niger* acid stable α-amylase, A. *niger* glucoamylase, *Rhizomucor miehei* lipase, A. *oryzae* alkaline protease, A. *oryzae* triose phosphate isomerase, or A. *nidulans* acetamidase. When a gene encoding an amylase is expressed in a bacterial species such as *E. coli,* a suitable promoter can be selected, for example, from a bacteriophage promoter including a T7 promoter and a phage lambda promoter. Examples of suitable promoters for the expression in a yeast species include but are not limited to the Gal 1 and Gal 10 promoters of *Saccharomyces cerevisiae* and the *Pichia pastoris* AOX1 or AOX2 promoters. For expression in *Trichoderma reesei,* the CBHII (cellobiohydrolase II) promoter may be used.

An expression vector may also comprise a suitable transcription terminator and, in eukaryotes, polyadenylation sequences operably linked to the DNA sequence encoding an α-amylase. Termination and polyadenylation sequences may suitably be derived from the same sources as the promoter.

The vector may further comprise a DNA sequence enabling the vector to replicate in the host cell. Examples of such sequences are the origins of replication of plasmids pUC19, pACYC177, pUB110, pE194, pAMB1, and pIJ702.

The vector may also comprise a selectable marker, *e.g.,* a gene the product of which complements a defect in the isolated host cell, such as the *dal* genes from *B*. *subtilis* or *B. licheniformis,* or a gene that confers antibiotic resistance such as, *e.g.,* ampicillin, kanamycin, chloramphenicol or tetracycline resistance. Furthermore, the vector may comprise *Aspergillus* selection markers such as *amdS, argB, niaD* and xxsC, a marker giving rise to hygromycin resistance, or the selection may be accomplished by co-transformation, such as known in the art. *See e.g.,* International PCT Application WO 91/17243.

As noted above, while intracellular expression or solid-state fermentation may be advantageous in some respects, *e.g*., when using certain bacteria or fungi as host cells, one aspect of the compositions and methods contemplates expression of an α-amylase into the culture medium.

In general, "full-length," "mature," or "precursor" amylases includes a signal sequence at the amino terminus that permits secretion into the culture medium. If desirable, this signal peptide may be replaced by a different sequence, conveniently accomplished by substitution of the DNA sequences encoding the respective signal polypeptide.

The expression vector typically includes the components of a cloning vector, such as, for example, an element that permits autonomous replication of the vector in the selected host organism and one or more phenotypically detectable markers for selection purposes. The expression vector normally comprises control nucleotide sequences such as a promoter, operator, ribosome binding site, translation initiation signal and optionally, a repressor gene or one or more activator genes. Additionally, the expression vector may comprise a sequence coding for an amino acid sequence capable of targeting the amylase to a host cell organelle such as a peroxisome, or to a particular host cell compartment. Such a targeting sequence includes but is not limited to the sequence, SKL. For expression under the direction of control sequences, the nucleic acid sequence of the amylase is operably linked to the control sequences in proper manner with respect to expression.

The procedures used to ligate the DNA construct encoding an amylase, the promoter, terminator and other elements, respectively, and to insert them into suitable vectors containing the information necessary for replication, are well known to persons skilled in the art (*see, e.g.,* Sambrook et al., MOLECULAR CLONING: A LABORATORY MANUAL, 2nd ed., Cold Spring Harbor, 1989, and 3rd ed., 2001).

An isolated cell, either comprising a DNA construct or an expression vector, is advantageously used as a host cell in the recombinant production of an amylase. The cell may be transformed with the DNA construct encoding the enzyme, conveniently by integrating the DNA construct (in one or more copies) in the host chromosome. This integration is generally considered to be an advantage, as the DNA sequence is more likely to be stably maintained in the cell. Integration of the DNA constructs into the host chromosome may be performed according to conventional methods, *e.g*., by homologous or heterologous recombination. Alternatively, the cell may be transformed with an expression vector as described above in connection with the different types of host cells.

Examples of suitable bacterial host organisms are Gram positive bacterial species such as *Bacillaceae* including *Bacillus subtilis, Bacillus licheniformis, Bacillus lentus, Bacillus brevis, Geobacillus* (formerly *Bacillus*) *stearothermophilus, Bacillus alkalophilus, Bacillus amyloliquefaciens, Bacillus coagulans, Bacillus lautus, Bacillus megaterium,* and *Bacillus thuringiensis; Streptomyces* species such as *Streptomyces murinus;* lactic acid bacterial species including *Lactococcus* sp. such as *Lactococcus lactis; Lactobacillus* sp. including *Lactobacillus reuteri*; *Leuconostoc* sp.; *Pediococcus* sp.; and *Streptococcus* sp. Alternatively, strains of a Gram negative bacterial species belonging to *Enterobacteriaceae* including *E. coli,* or to *Pseudomonadaceae* can be selected as the host organism.

A suitable yeast host organism can be selected from the biotechnologically relevant yeasts species such as but not limited to yeast species such as *Pichia* sp., *Hansenula* sp., or *Kluyveromyces, Yarrowinia, Schizosaccharomyces* species or a species of *Saccharomyces,* including *Saccharomyces cerevisiae* or a species belonging to *Schizosaccharomyces* such as, for example, *S*. *pombe* species. A strain of the methylotrophic yeast species, *Pichia pastoris,* can be used as the host organism. Alternatively, the host organism can be a *Hansenula* species. Suitable host organisms among filamentous fungi include species of *Aspergillus, e.g., Aspergillus niger, Aspergillus oryzae, Aspergillus tubigensis, Aspergillus awamori,* or *Aspergillus nidulans.* Alternatively, strains of a *Fusarium* species, *e.g., Fusarium oxysporum* or of a *Rhizomucor* species such as *Rhizomucor miehei* can be used as the host organism. Other suitable strains include *Thermomyces* and *Mucor* species. In addition, *Trichoderma reesei* can be used as a host. A suitable procedure for transformation of *Aspergillus* host cells includes, for example, that described in EP 238023.

In a yet further aspect, a method of producing an α-amylase is provided comprising cultivating a host cell as described above under conditions conducive to the production of the enzyme and recovering the enzyme from the cells and/or culture medium.

The medium used to cultivate the cells may be any conventional medium suitable for growing the host cell in question and obtaining expression of an amylase. Suitable media and media components are available from commercial suppliers or may be prepared according to published recipes *(e.g.,* as described in catalogues of the American Type Culture Collection).

In one aspect, an enzyme secreted from the host cells is used in a whole broth preparation. In the present methods, the preparation of a spent whole fermentation broth of a recombinant microorganism can be achieved using any cultivation method known in the art resulting in the expression of an alpha-amylase. Fermentation may, therefore, be understood as comprising shake flask cultivation, small- or large-scale fermentation (including continuous, batch, fed-batch, or solid state fermentations) in laboratory or industrial fermenters performed in a suitable medium and under conditions allowing the amylase to be expressed or isolated. The term "spent whole fermentation broth" is defined herein as unfractionated contents of fermentation material that includes culture medium, extracellular proteins (*e.g*., enzymes), and cellular biomass. It is understood that the term "spent whole fermentation broth" also encompasses cellular biomass that has been lysed or permeabilized using methods well known in the art.

An enzyme secreted from the host cells may conveniently be recovered from the culture medium by well-known procedures, including separating the cells from the medium by centrifugation or filtration, and precipitating proteinaceous components of the medium by means of a salt such as ammonium sulfate, followed by the use of chromatographic procedures such as ion exchange chromatography, affinity chromatography, or the like.

An aspect contemplates the polynucleotide in a vector is operably linked to a control sequence that is capable of providing for the expression of the coding sequence by the host cell, *i.e.* the vector is an expression vector. The control sequences may be modified, for example by the addition of further transcriptional regulatory elements to make the level of transcription directed by the control sequences more responsive to transcriptional modulators. The control sequences may in particular comprise promoters.

Host cells may be cultured under suitable conditions that allow expression of an amylase. Expression of the enzymes may be constitutive such that they are continually produced, or inducible, requiring a stimulus to initiate expression. In the case of inducible expression, protein production can be initiated when required by, for example, addition of an inducer substance to the culture medium, for example dexamethasone or IPTG or Sopharose. Polypeptides can also be produced recombinantly in an *in vitro* cell-free system, such as the TNT^{™} (Promega) rabbit reticulocyte system.

An amylase-expressing host also can be cultured in the appropriate medium for the host, under aerobic conditions. Shaking or a combination of agitation and aeration can be provided, with production occurring at the appropriate temperature for that host, *e.g.,* from about 25°C to about 75°C *(e.g.,* 30°C to 45°C), depending on the needs of the host and production of the desired amylase. Culturing can occur from about 12 to about 100 hours or greater (and any hour value there between, *e.g*., from 24 to 72 hours). Typically, the culture broth is at a pH of about 5.5 to about 8.0, again depending on the culture conditions needed for the host relative to production of an amylase.

### 5.2. Materials and Methods for Protein Purification

Fermentation, separation, and concentration techniques are well-known in the art and conventional methods can be used in order to prepare a concentrated amylase polypeptide-containing solution.

After fermentation, a fermentation broth is obtained, the microbial cells and various suspended solids, including residual raw fermentation materials, are removed by conventional separation techniques in order to obtain an amylase solution. Filtration, centrifugation, microfiltration, rotary vacuum drum filtration, ultrafiltration, centrifugation followed by ultrafiltration, extraction, or chromatography, or the like, are generally used.

It is desirable to concentrate an α-amylase polypeptide-containing solution in order to optimize recovery. Use of unconcentrated solutions requires increased incubation time in order to collect the purified enzyme precipitate.

The enzyme containing solution is concentrated using conventional concentration techniques until the desired enzyme level is obtained. Concentration of the enzyme containing solution may be achieved by any of the techniques discussed herein. Exemplary methods of purification include but are not limited to rotary vacuum filtration and/or ultrafiltration.

The enzyme solution is concentrated into a concentrated enzyme solution until the enzyme activity of the concentrated amylase polypeptide-containing solution is at a desired level.

Concentration may be performed using, *e.g.,* a precipitation agent, such as a metal halide precipitation agent. Metal halide precipitation agents include but are not limited to alkali metal chlorides, alkali metal bromides and blends of two or more of these metal halides. Exemplary metal halides include sodium chloride, potassium chloride, sodium bromide, potassium bromide and blends of two or more of these metal halides. The metal halide precipitation agent, sodium chloride, can also be used as a preservative.

The metal halide precipitation agent is used in an amount effective to precipitate the α-amylase polypeptide. The selection of at least an effective amount and an optimum amount of metal halide effective to cause precipitation of the enzyme, as well as the conditions of the precipitation for maximum recovery including incubation time, pH, temperature and concentration of enzyme, will be readily apparent to one of ordinary skill in the art, after routine testing.

Generally, at least about 5% w/v (weight/volume) to about 25% w/v of metal halide is added to the concentrated enzyme solution, and usually at least 8% w/v. Generally, no more than about 25% w/v of metal halide is added to the concentrated enzyme solution and usually no more than about 20% w/v. The optimal concentration of the metal halide precipitation agent will depend, among others, on the nature of the specific amylase polypeptide and on its concentration in the concentrated enzyme solution.

Another alternative to effect precipitation of the enzyme is to use organic compounds. Exemplary organic compound precipitating agents include: 4-hydroxybenzoic acid, alkali metal salts of 4-hydroxybenzoic acid, alkyl esters of 4-hydroxybenzoic acid, and blends of two or more of these organic compounds. The addition of said organic compound precipitation agents can take place prior to, simultaneously with or subsequent to the addition of the metal halide precipitation agent, and the addition of both precipitation agents, organic compound and metal halide, may be carried out sequentially or simultaneously.

Generally, the organic precipitation agents are selected from the group consisting of alkali metal salts of 4-hydroxybenzoic acid, such as sodium or potassium salts, and linear or branched alkyl esters of 4-hydroxybenzoic acid, wherein the alkyl group contains from 1 to 12 carbon atoms, and blends of two or more of these organic compounds. The organic compound precipitation agents can be, for example, linear or branched alkyl esters of 4-hydroxybenzoic acid, wherein the alkyl group contains from 1 to 10 carbon atoms, and blends of two or more of these organic compounds. Exemplary organic compounds are linear alkyl esters of 4-hydroxybenzoic acid, wherein the alkyl group contains from 1 to 6 carbon atoms, and blends of two or more of these organic compounds. Methyl esters of 4-hydroxybenzoic acid, propyl esters of 4-hydroxybenzoic acid, butyl ester of 4-hydroxybenzoic acid, ethyl ester of 4-hydroxybenzoic acid and blends of two or more of these organic compounds can also be used. Additional organic compounds also include but are not limited to 4-hydroxybenzoic acid methyl ester (named methyl PARABEN), 4-hydroxybenzoic acid propyl ester (named propyl PARABEN), which also are both amylase preservative agents. For further descriptions, *see, e.g.,* U.S. Patent No. 5,281,526.

Addition of the organic compound precipitation agent provides the advantage of high flexibility of the precipitation conditions with respect to pH, temperature, amylase polypeptide concentration, precipitation agent concentration, and time of incubation.

The organic compound precipitation agent is used in an amount effective to improve precipitation of the enzyme by means of the metal halide precipitation agent. The selection of at least an effective amount and an optimum amount of organic compound precipitation agent, as well as the conditions of the precipitation for maximum recovery including incubation time, pH, temperature and concentration of enzyme, will be readily apparent to one of ordinary skill in the art, in light of the present disclosure, after routine testing.

Generally, at least about 0.01% w/v of organic compound precipitation agent is added to the concentrated enzyme solution and usually at least about 0.02% w/v. Generally, no more than about 0.3% w/v of organic compound precipitation agent is added to the concentrated enzyme solution and usually no more than about 0.2% w/v.

The concentrated polypeptide solution, containing the metal halide precipitation agent, and the organic compound precipitation agent, can be adjusted to a pH, which will, of necessity, depend on the enzyme to be purified. Generally, the pH is adjusted at a level near the isoelectric point of the amylase. The pH can be adjusted at a pH in a range from about 2.5 pH units below the isoelectric point (pI) up to about 2.5 pH units above the isoelectric point.

The incubation time necessary to obtain a purified enzyme precipitate depends on the nature of the specific enzyme, the concentration of enzyme, and the specific precipitation agent(s) and its (their) concentration. Generally, the time effective to precipitate the enzyme is between about 1 to about 30 hours; usually it does not exceed about 25 hours. In the presence of the organic compound precipitation agent, the time of incubation can still be reduced to less about 10 hours and in most cases even about 6 hours.

Generally, the temperature during incubation is between about 4°C and about 50°C. Usually, the method is carried out at a temperature between about 10°C and about 45°C (*e.g.,* between about 20°C and about 40°C). The optimal temperature for inducing precipitation varies according to the solution conditions and the enzyme or precipitation agent(s) used.

The overall recovery of purified enzyme precipitate, and the efficiency with which the process is conducted, is improved by agitating the solution comprising the enzyme, the added metal halide and the added organic compound. The agitation step is done both during addition of the metal halide and the organic compound, and during the subsequent incubation period. Suitable agitation methods include mechanical stirring or shaking, vigorous aeration, or any similar technique.

After the incubation period, the purified enzyme is then separated from the dissociated pigment and other impurities and collected by conventional separation techniques, such as filtration, centrifugation, microfiltration, rotary vacuum filtration, ultrafiltration, press filtration, cross membrane microfiltration, cross flow membrane microfiltration, or the like. Further purification of the purified enzyme precipitate can be obtained by washing the precipitate with water. For example, the purified enzyme precipitate is washed with water containing the metal halide precipitation agent, or with water containing the metal halide and the organic compound precipitation agents.

During fermentation, an α-amylase polypeptide accumulates in the culture broth. For the isolation and purification of the desired amylase, the culture broth is centrifuged or filtered to eliminate cells, and the resulting cell-free liquid is used for enzyme purification. In one embodiment, the cell-free broth is subjected to salting out using ammonium sulfate at about 70% saturation; the 70% saturation-precipitation fraction is then dissolved in a buffer and applied to a column such as a Sephadex G-100 column, and eluted to recover the enzyme-active fraction. For further purification, a conventional procedure such as ion exchange chromatography may be used.

Purified enzymes are useful for laundry and cleaning applications. For example, they can be used in laundry detergents and spot removers. They can be made into a final product that is either liquid (solution, slurry) or solid (granular, powder).

A more specific example of purification, is described in Sumitani, J. et al. (2000) "New type of starch-binding domain: the direct repeat motif in the C-terminal region of Bacillus sp. 195 α-amylase contributes to starch binding and raw starch degrading," Biochem. J. 350: 477-484, and is briefly summarized here. The enzyme obtained from 4 liters of a *Streptomyces lividans* TK24 culture supernatant was treated with (NH₄)₂SO₄ at 80% saturation. The precipitate was recovered by centrifugation at 10,000 x g (20 min. and 4°C) and re-dissolved in 20 mM Tris/HCl buffer (pH 7.0) containing 5 mM CaCl₂. The solubilized precipitate was then dialyzed against the same buffer. The dialyzed sample was then applied to a Sephacryl S-200 column, which had previously been equilibrated with 20 mM Tris/HCl buffer, (pH 7.0), 5 mM CaCl₂, and eluted at a linear flow rate of 7 mL/hr with the same buffer. Fractions from the column were collected and assessed for activity as judged by enzyme assay and SDS-PAGE. The protein was further purified as follows. A Toyopearl HW55 column (Tosoh Bioscience, Montgomeryville, PA; Cat. No. 19812) was equilibrated with 20 mM Tris/HCl buffer (pH 7.0) containing 5 mM CaCl₂ and 1.5 M (NH₄)₂SO₄. The enzyme was eluted with a linear gradient of 1.5 to 0 M (NH₄)₂SO₄ in 20 mM Tris/HCL buffer, pH 7.0 containing 5 mM CaCl₂. The active fractions were collected, and the enzyme precipitated with (NH₄)₂SO₄ at 80% saturation. The precipitate was recovered, re-dissolved, and dialyzed as described above. The dialyzed sample was then applied to a Mono Q HR5/5 column (Amersham Pharmacia; Cat. No. 17-5167-01) previously equilibrated with 20 mM Tris/HCl buffer (pH 7.0) containing 5 mM CaCl₂, at a flow rate of 60 mL/hour. The active fractions are collected and added to a 1.5 M (NH₄)₂SO₄ solution. The active enzyme fractions were re-chromatographed on a Toyopearl HW55 column, as before, to yield a homogeneous enzyme as determined by SDS-PAGE. *See* Sumitani, J. et al. (2000) Biochem. J. 350: 477-484, for general discussion of the method and variations thereon.

For production scale recovery, an amylase polypeptide can be partially purified as generally described above by removing cells via flocculation with polymers. Alternatively, the enzyme can be purified by microfiltration followed by concentration by ultrafiltration using available membranes and equipment. However, for some applications, the enzyme does not need to be purified, and whole broth culture can be lysed and used without further treatment. The enzyme can then be processed, for example, into granules.

### 6. Compositions comprising variants α-amylases

Also described are compositions comprising one or more of the described α-amylase variants. Such compositions include, for example, enzyme concentrates, enzyme blends, purified enzyme preparations, partially purified enzyme products, clarified broth products, whole broth products, food additives, and cleaning products. The compositions can be provided in a variety of physical forms including liquids, slurries, gels, cakes, powders, granules, and the like. The compositions can be lyophilized, concentrated, frozen, spray-dried, or otherwise processed in a variety of known or useful manners. The compositions can be provided in standard sizes for certain commercial applications, or custom packaged, or provided in bulk containers of any type.

The compositions may further comprise or be used in conjunction with any number of formulation ingredients, such as buffers, salts, chelators, preservatives, anti-microbials, polymers, bulking agents, and the like. Especially where the compositions are cleaning compositions, they may further comprising, *e.g*., surfactants, oxidants, chelators, or other cleaning agents. Exemplary compositions are laundry detergents and dishwashing detergents, including automatic dishwashing detergents. The compositions may further comprise or be used in conjunction with one or more additional polypeptides. The one or more additional polypeptides may include at least one enzyme. Additional enzymes include but are not limited to other α-amylases, β-amylases, glucoamylases, isoamylases, isomerases, phytases, proteases, cellulases, lignases, hemicellulases, lipases, phospholipases, and cutinases.

Also described herein are compositions comprising one or more of the present variant amylases, or enzyme blends further comprising one or more additional enzymes, are useful for liquefaction starch. In some compositions described herein, the compositions are useful to facilitate removal of starch from textiles, paper, glass, plastic, metal, canvas, porcelain, and other surfaces. In some compositions described herein, the compositions are prepared or formulated for use as food additives or processing aids suitable for use in food processes. The compositions are particularly effective where at least a portion of the starch is in the form of starch bundles.

### 8. Methods of use

Also described herein are methods for using the described variant α-amylases or compositions comprising such variants. In some methods described herein, the variant α-amylases are used in a method for liquefying a complex carbohydrate, such as a starch slurry, particularly where the starch slurry contains starch bundles. The method generally comprises making a slurry comprising the complex carbohydrate, heating the slurry to an acceptable temperature for liquefaction, adding a composition comprising at least one α-amylase variant as described herein, to the slurry, and incubating the slurry with the composition for a time and at a temperature sufficient to liquefy the complex carbohydrate. As used herein "liquefy" does not mean that every available substrate linkage is cleaved, rather it means that the complex carbohydrate is at least partially hydrolyzed, as evidenced by a measurable reduction in final viscosity, an increase in the DE of the slurry, the release of low DP fragments/products, or another measure of an increase in reducing groups, dextrins, or α-maltose units.

The temperature of liquefaction can range from room temperature to over 100°C, but is more preferably is about 50°C to about 95°C. Liquefaction can entail the use of a complex temperature curve over time, for example, the reaction may start at a low temperature and be increased by methods known in the art to the desired end temperature. The temperature may also be reduced after a specific amount of time, or after a desired end-point in reached in terms of viscosity, DE value, or another measure of liquefaction. The skilled artisan will thus appreciate that the method need not entail a specific temperature for a particular duration, provided that the amylase activity can function at the temperature and under the conditions provided. Other conditions that impact the activity include the pH and the calcium ion concentration, in addition to the presence or absence of inhibitors or the like.

The slurry may include about 20-40% starch on a dry-weight basis, for example, between about 30 to about 36 or 37.5% starch. Lower amounts of starch can be used but are generally not economical. Maximum viscosity and related factors, such as required power inputs for mixing may limit the maximum amount of starch to be used in the slurry. The skilled artisan will appreciate the practical considerations in making the starch slurry. The skilled artisan will appreciate that, the more the viscosity is reduced, the further the starch is liquefied, the greater the production of dextrins (or the higher the DE of the resultant liquefied starch). Thus, the peak viscosity is reduced by at least 10, 20, 25, 30, 40, or even 50% or more relative to the peak viscosity of a comparable slurry liquefied by a wild-type enzyme from which the variant was derived, or treated with a currently-available commercial enzyme preparation.

In some methods described herein, the liquefaction is part of a fermentation to produce, *e.g.,* a food product, a food additive, a fuel, a fuel additive, and the like. As described herein, fermentation produces a fuel or fuel additive such as an alcohol, for example, ethanol, butanol, or another lower alcohol. Described herein are methods for producing ethanol using the variant α-amylases, compositions, or enzyme blends described herein. According to such methods, following addition of the variant enzyme, the resultant liquefied starch slurry is fermented with one or more organisms capable of producing ethanol, under conditions and for a time suitable for the production of ethanol in the fermentation. Alternatively, the variant amylase and organisms are present at the same time, as in the case of simultaneous saccharification and fermentation (SSF).

Also described herein are methods of cleaning a surface to remove an unwanted or undesired starch residue, particularly where at least a portion of the starch is in the form of starch bundles. The methods comprise the steps of providing a surface that has a starch residue to be removed, contacting the surface with a composition that comprises one or more variant α-amylases, for a time and at a temperature sufficient, and under conditions permissive to result in removal of the starch residue. The surface can be on any material; for example, it can be on a dish, plate, glass, etc, or it can be on clothing or fabric. It can also be for example a counter-top or work surface, or a commercial vessel of any type that must be periodically or regularly cleaned.

Also described herein are methods of treating a woven material using the α-amylase variants described herein. Methods of treating woven materials, such as fabrics, with amylases are known in the art, and encompass what is referred to as "desizing." The methods comprise contacting the woven material with a liquid comprising an α-amylase variant or a composition comprising a variant in accordance herewith. In some methods described herein, the woven material is a fabric or textile. In another method described herein, the woven material is treated with the liquid under pressure. The liquid is generally an aqueous solution. The α-amylase variants described herein can be used alone or with other desizing chemical reagents, such as detergents and/or desizing enzymes to desize woven materials such as fabrics, including cotton and cotton-containing fabrics.

The working examples provided below are provided to further describe and illustrate certain aspects of the variants of the α-amylases, and thus should not be construed to be limiting.

### EXAMPLES

### Example 1. Purification of uPWA

A variant α-amylase derived from *Pyrococcus woesei,* uPWA (SEQ ID NO: 2 in U.S. Patent No. 7,273,740), was purchased from Verenium (San Diego, CA, USA). uPWA contains 58 amino acid substitutions relative to the wild-type *P. woesei* amylase, PWA. The amino acid sequences of uPWA and PWA are shown, below, as SEQ ID NO: 5 and SEQ ID NO: 1, respectively:
Variant *P. woesei* amylase, uPWA (SEQ ID NO: 5) 
AKYSELEKGG VIMQAFYWDV PSGGIWWDTI RQKIPEWYDA GISAIWIPPA
SKGMGGAYSM GYDPYDFFDL GEYDQKGTVE TRFGSKQELV NMINTAHAYG
MKVIADIVIN HRAGGDLEWN PFVNDYTWTD FSKVASGKYT ANYLDFHPNE
LHAGDSGTFG GYPDICHDKS WDQYWLWASQ ESYAAYLRSI GIDAWRFDYV
KGYAPWVVKD WLNWWGGWAV GEYWDTNVDA VLNWAYSSGA KVFDFALYYK
MDEAFDNKNI PALVSALQNG QTVVSRDPFK AVTFVANHDT DIIWNKYPAY
AFILTYEGQP TIFYRDYEEW LNKDKLKNLI WIHENLAGGS TDIVYYDNDE
LIFVRNGYGD KPGLITYINL GSSKAGRWVY VPKFAGACIH EYTGNLGGWV
DKYVYSSGWV YLEAPAYDPA NGQYGYSVWS YCGVG
Wild-type *P. woesei* amylase (SEQ ID NO: 1)

AKYLELEEGG VIMQAFYWDV PGGGIWWDHI RSKIPEWYEA GISAIWLPPP
SKGMSGGYSM GYDPYDYFDL GEYYQKGTVE TRFGSKEELV RLIQTAHAYG
IKVIADVVIN HRAGGDLEWN PFVGDYTWTD FSKVASGKYT ANYLDFHPNE
LHCCDEGTFG GFPDICHHKE WDQYWLWKSN ESYAAYLRSI GFDGWRFDYV
KGYGAWVVRD WLNWWGGWAV GEYWDTNVDA LLSWAYESGA KVFDFPLYYK
MDEAFDNNNI PALVYALQNG QTVVSRDPFK AVTFVANHDT DIIWNKYPAY
AFILTYEGQP VIFYRDFEEW LNKDKLINLI WIHDHLAGGS TTIVYYDNDE
LIFVRNGDSR RPGLITYINL SPNWVGRWVY VPKFAGACIH EYTGNLGGWV
DKRVDSSGWV YLEAPPHDPA NGYYGYSVWS YCGVG

uPWA was purified by hydrophobic interaction chromatography and transferred to a high pH buffer consisting of 50 mM Glycine, pH 10 and 2 mM CaCl₂, and then mix for twenty minutes. Ammonium sulfate was added to a 1M final concentration and the solution was allowed to mix for another 30 minutes, and then applied to a 30 mL Phenyl Sepharose column equilibrated with the same buffer. The column was washed with the same buffer until a stable base line was reached. A 300 mL gradient was applied to reach 50 mM Glycine, pH 10, 2 mM CaCl₂, followed by 300 mL wash in the same buffer. Finally the protein was eluted in 50 mM Glycine, pH 10, 2 mM calcium chloride, 40% propylene glycol.

Pooled fractions were concentrated in a Vivaspin concentrator (MWCO 10,000) to a final protein concentration of 20 mg/mL. The purified protein was stored in elution buffer (50 mM Glycine, pH 10, 2 mM CaCl₂, 40% propylene glycol) at 4 °C. The concentration was determined by optical density at 280 nm, and SDS-PAGE was used to evaluate purity. Prior to crystallization, the protein buffer storage buffer was exchanged to water using a Vivaspin concentrator (MWCO 10,000) and the final protein concentration was adjusted to 10 mg/ml.

### Example 2. Crystallization of uPWA

Crystallization experiments were carried out at room temperature using 24-well sitting drops in Cryschem plates (Hampton Research). Initial screening was carried out using commercial available screens (Hampton and Qiagen). Drops were mixed as 2 µl protein sample with an equal amount of reservoir solution and left to equilibrate against 250 µl reservoir solutions. Crystals appeared after 5-7 days with reservoir conditions consisting of 0.1M Tris-HCl pH 8.5 and 20% (v/v) Ethanol or 0.2 M NaCl, 0.1 M HEPES pH 7.5 and 10% (v/v) isopropanol. Prior to X-ray diffraction analysis, crystals were cryoprotected by the addition of grains of sucrose to the drop, which were allowed to dissolve. The crystals were mounted in nylon loops and flash-cooled in liquid nitrogen.

Data were collected using synchrotron radiation at the Stanford Synchrotron Radiation Lightsource (SSRL beamline BL12-2). A full dataset was collected, integrated and scaled at 50 - 2.1 Å resolution with XDS (Kabsch, W. (1993) J. Appl. Crystallogr. 26:795-800).

### Example 3. uPWA structure determination and refinement

The structure of uPWA was determined by molecular replacement using Phaser (McCoy, A. J. et al. (2007) J. Appl. Crystallogr. 40:658-674) with the structure of the wild-type *P. woesei* amylase (PDB access code: 1MWO; Linden, A. et al. (2003) J. Biol. Chem. 278:9875-9884) as a search model. Model refinement, map interpretation, and model building were performed using PHENIX (Adams, P. D. et al. (2002) Acta Crystallogr. D Biol. Crystallogr. 58:1948-1954) and COOT (Emsley, P. and Cowtan, K. (2004) Acta Crystallogr. D Biol. Crystallogr. 60:2126-2132). Model refinement statistics are listed in Table 1.

**Table 1. Model refinement statistics**

| | |
|---|---|
| Resolution range (A) | 50 Å - 2.1 Å |
| Modelled | 435 amino acid, 191 waters, 2 sucrose, 4 Ca²⁺, 1 Tris⁺, 1 Zn²⁺, 1 SO₄²⁻ and 1 β-cyclodextrin |
| R_{work} (%)¹ | 16.5% |
| R_{free} (%)² | 20.8% |
| Average B-factor (Å) | 30.8 |
| RMSD_{bonds} (Å)³ | 0.008 |
| RMSD_{angels} (Å)³ | 1.25 |
| ¹R_{work} (%) = 100×∑ₕₖₗ\| Fobs- Fcalc\| / ∑ₕₖₗ Fobs where Fobs and Fcalc are the observed and calculated structure factors respectively. ²R_{free}(%) is calculated using a randomly selected 5% sample of reflection data omitted from the refinement. ³Root mean square deviation from ideal bond lengths and angels. | |

The structure was determined to a resolution of 2.1 Å. The final model encompassed residues 1 to 435 of the polypeptide, 2 sucrose molecules, bound ions (4 Ca²⁺, 1 Zn²⁺, 1 Tris⁺, 1 SO₄²⁻), 191 water molecules, and 1 β-cyclodextrin molecule and yields R_{work} and R_{free} values of 16.5% and 20.8%, respectively, at 50 - 2.1 Å resolution and with 94.2% of residues in the most favored regions of the Ramachandran plot (Chen, V. B. et al. (2010) Acta Crystallogr. Sect. D-Biol. Crystallogr. 66:12-21).

The uPWA structure displayed the canonical fold of the CAZy glycosylhydrolase 13 family with characteristic A, B and C domains (Figure 1). The secondary structure is shown in Figure 2. Despite the presence of 58 mutations with respect to the wild-type *P. woesei* amylase, the overall structure was similar to the previously-described structure (wtPWA, PDB access code: 1MWO; Linden, A. et al. (2003) J. Biol. Chem. 278:9875-9884), with a root mean square deviation of 0.6 Å on the C^{α} backbone positions. The central A domain (residues 1-109 and residues 170-340) retains the characteristic TIM barrel fold and harbors the active site residues Asp²⁸⁹, Glu²²², and Asp¹⁹⁸ (Banner, D. W. et al. (1975) Nature 255:609-614). The relatively small B domain constitutes part of the substrate binding cleft and possesses a Ca,Zn-metal center (Linden, A. et al. (2003) J. Biol. Chem. 278:9875-9884). The C-terminal C domain (residues 341-435) consists of an eight-stranded antiparallel β-sheet including the classical Greek key motif. In addition to the Ca²⁺ and Zn²⁺ ions in the unique metal center, three more Ca²⁺ sites were found: two within the A domain and one in the C domain, all corresponding to sites previously reported for wtPWA with and without α-acarbose (PDB access codes 1MXD and 1MWO, respectively), or Mg²⁺ sites for wtPWA α-acarbose (PDB access code: 1MXO).

A strong anomalous peak was observed at the Zn binding site, whereas no peaks were observed at any of the other binding sites. Surprisingly, a tightly bound cyclodextrin was found at the N-terminus of the A domain (Figure 1). The binding site is formed by a small helix preceding the first β-strand, the two loops of helix 6/strand 7 and helix 7/strand 8, and the loop connecting the A and C domains. Part of the cyclodextrin binding site overlaps with the α-acarbose binding site (Ac-II) observed in the wild-type polypeptide. A sulfate ion is located at the center of the cyclodextrin ring.

Furthermore, a sucrose molecule is bound between the two loops of helix 4/strand 5 and helix 5/strand 6, in close vicinity of the cyclodextrin. A second sucrose molecule is found close to helix 3b and the C-terminal part of domain B, overlapping with the methyl tetraglycol binding site of the wild-type polypeptide with α-acarbose (PDB access code: 1MXD).

None of the 58 amino acid substitutions in uPWA involve residues in direct contact with metal ions or other ligands; however, C153A, C154G, E156S and H168D are near the Ca,Zn-metal center and D358Y, S359G, R360D, R361K and P372S are close to the bound cyclodextrin. The majority of the substitutions are located on the surface of the enzyme.

### Example 4. Correlation between SEL winners and groove positions

A full, 488-position, site evaluation library (SEL) was prepared for screening variants of a *Cytophaga* sp. amylase (AAF00567.1, GI# 6006681; SEQ ID NO: 4) having a deletion in the starch-binding loop (ΔR178+ΔG179; underlined in SEQ ID NO: 4).
*Cytophaga* sp. α-amylase (AAF00567.1, GI# 6006681; SEQ ID NO: 4):

AATNGTMMQY FEWYVPNDGQ QWNRLRTDAP YLSSVGITAV WTPPAYKGTS
QADVGYGPYD LYDLGEFNQK GTVRTKYGTK GELKSAVNTL HSNGIQVYGD
VVMNHKAGAD YTENVTAVEV NPSNRNQETS GEYNIQAWTG FNFPGRGTTY
SNFKWQWFHF DGTDWDQSRS LSRIFKFRGT GKAWDWEVSS ENGNYDYLMY
ADIDYDHPDV VNEMKKWGVW YANEVGLDGY RLDAVKHIKF SFLKDWVDNA
RAATGKEMFT VGEYWQNDLG ALNNYLAKVN YNQSLFDAPL HYNFYAASTG
GGYYDMRNIL NNTLVASNPT KAVTLVENHD TQPGQSLEST VQPWFKPLAY
AFILTRSGGY PSVFYGDMYG TKGTTTREIP ALKSKIEPLL KARKDYAYGT
QRDYIDNPDV IGWTREGDST KAKSGLATVI TDGPGGSKRM YVGTSNAGEI
WYDLTGNRTD KITIGSDGYA TFPVNGGSVS VWVQQ

Such SELs are known in the art, and are routinely used to evaluate the effect of individual substitutions, or combinations of mutations, in polypeptides. The variant Cytophaga amylase polypeptides were scored for performance based on their ability to hydrolyze corn starch. The ratio of the activity of a variant to the activity of the wild-type Cytophaga sp. amylase, normalized for expression, was referred to as the performance index (PI) for activity. Outright SEL "winners" were variants that had a PI for activity >1, indicating increased activity on corn starch. However, mutations that result in PI activity <1 can often provide performance benefits in combination with other mutations. The ratio of the level of expression of a variant and the level of expression of the wild-type *Cytophaga* sp. amylase was referred to as PI for expression. When analyzing SEL data, it is often desirable to exclude data from variants having a PI for expression below a preselected threshold *(e.g.,* <0.3) to avoid errors that arise from measuring low levels of enzyme activity.

Figure 6 is a histogram showing the distribution of PI for activity values of all SEL variants having a PI for expression >0.3. The X-axis indicates PI for activity and the Y-axis indicates the number of variants having the corresponding PI for activity. Figure 7 is a histogram showing the distribution of PI for activity values of SEL variants having substitutions only in groove positions *(i.e.,* positions 1, 2, 3, 4, 38, 88, 91, 92, 93, 94, 95, 96, 97, 230, 251, 252, 253, 254, 255, 256, 308, 314, 315, 316, 317, 318, 319, 320, 354, 355, 356, 357, 358, 359, 396, 397, 398, 399, 400, 401, 402, and 403, referring to SEQ ID NO: 4) and having a PI for expression >0.3. The distribution of groove position variants is more Gaussian, with fewer variants having a PI for activity <1. These data indicate that mutations at groove positions are more likely to produce a variant amylase with a PI for activity >0.8, >0.9, and >1, and less likely to produce a variant amylase with a PI for activity <1, <0.9, and <0.8, compared to a mutation made indiscriminately, *i.e.,* anywhere in an amylase polypeptide from position 1 to 488.

### Example 5. Correlation between groove positions and activity on starch substrates

Individual variants obtained from the SEL library of Example 4 were tested for expression, activity on an amylose substrate, and activity on an amylopectin substrate. Amylose is an unbranched component of starch consisting of glucose residues in α-1,4 linkages. The starch molecules in amylose are elongated and resemble the starch molecules found in starch bundles. Amylopectin is a branched component of starch, consisting of α-1,6 linkages and α-1,4 linkages.

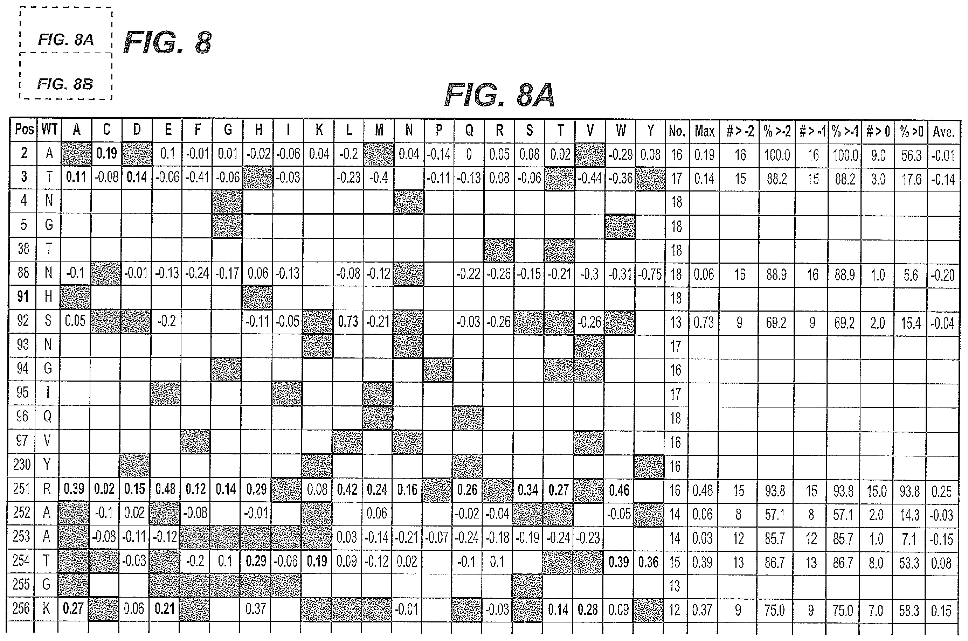

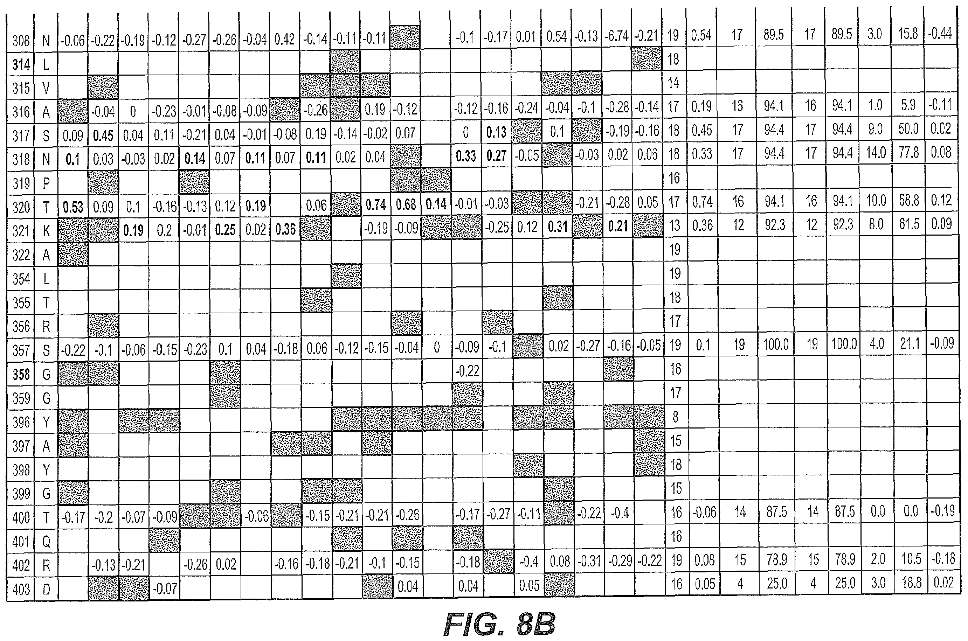

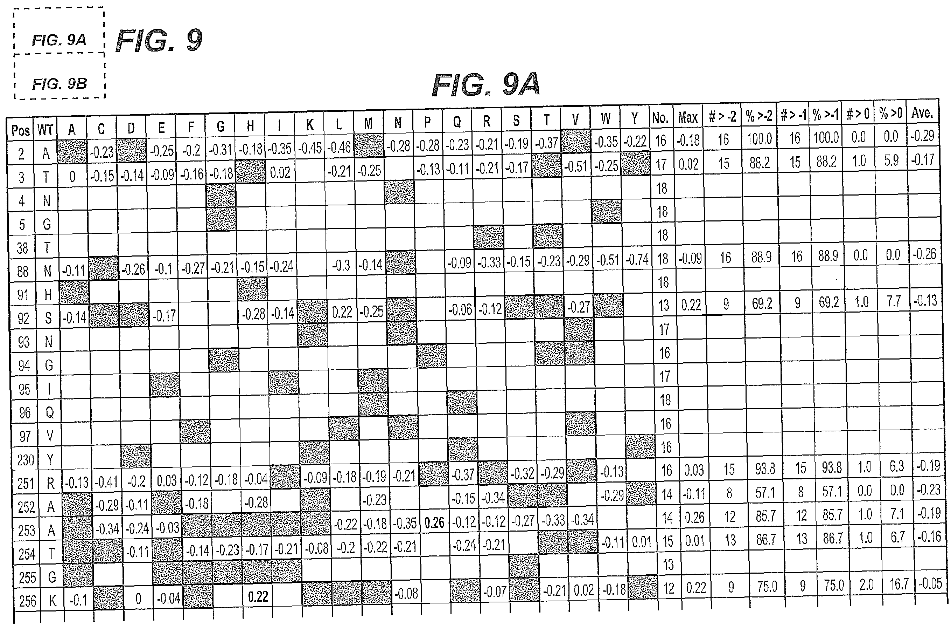

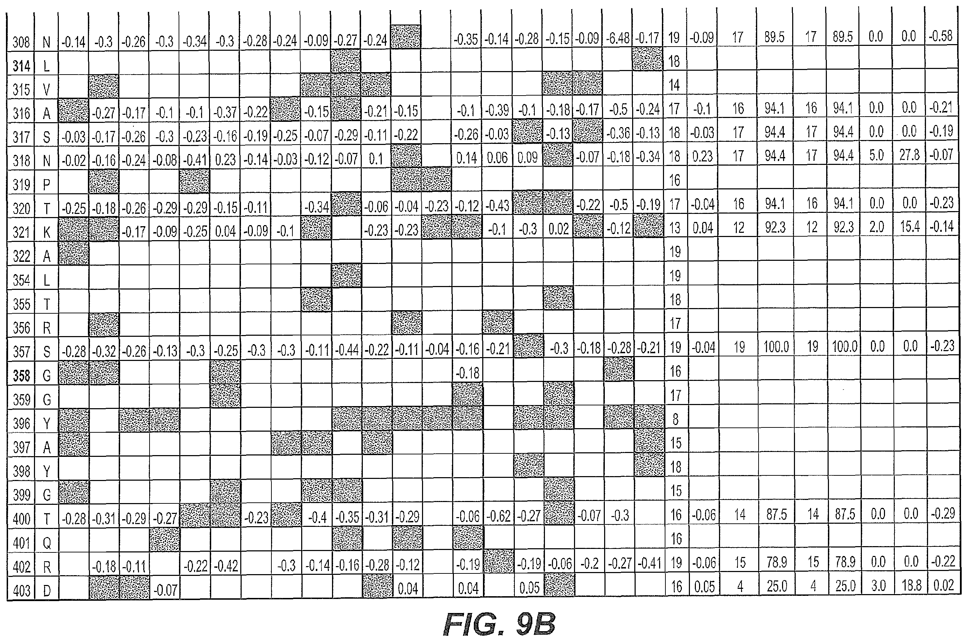

The results of the assays are shown in Figures 8 and 9. The amino acid position numbers (Pos) and wild-type residues present at those positions (WT) are shown in columns 1 and 2, respectively. The amino acid substitutions present at the indicated position in the tested variants are shown in the adjacent 20 columns. Grey highlighting in the cells indicates that a variant was not made. Blank cells indicate that a variant did not express at sufficient levels to permit further analyses. Variants with substitutions at positions 2, 3, 88, 92, 251, 252, 253, 254, 256, 308, 316, 317, 318, 320, 321, 357, 400, 402, and 404 (referring to SEQ ID NO: 4) were tested for activity on an amylose substrate (Figure 8) and amylopectin substrate (Figure 9).

The data values are reported as log (2) of Performance Index (PI), in which case a negative number indicates decreased activity compared to an amylase with the wild-type amino acid residue at the indicated position and a positive number indicates increases activity compared to an amylase with the wild-type amino acid residue at the indicated position, and where each unit change represents a factor of 2. Data for mutations that increase the activity on the substrate are shown in bold font. The number of variants tested (No.), maximum log(2) PI values (Max), number of variants with log(2) PI greater than -2 (# <-2), percentage of variants with log(2) PI greater than 2 (% >-2), number of variants with log(2) PI greater than -1 (# <-1), percentage of variants with log(2) PI greater than -1 (% >-1), number of variants with log(2) PI greater than 0 (# <0), percentage of variants with log(2) PI greater than 0 (% >0), and average log(2) PI values (Ave.) are indicated in subsequent columns.

Substitutions at positions 92, 251, 254, 256, 317, 318, 320, and 321 (referring to SEQ ID NO: 4), improved activity on the amylose substrate. Substitutions at positions 253 and 256 improved activity on the amylose substrate. Particular substitutions that improved activity on the amylose substrate were S92L, R251A, R251C, R251D, R251E, R251F, R251G, R251H, R251L, R251M, R251N, R251Q, R251S, R251T, and R251W, T254H, T254K, T254W, and T254Y, K256A, K256E, K256T, and K256V, S317C and S317R, N318A N318F, N318H, N318K, N318Q, and N318R, T320A, T320H, T320M, T320N, and T320P, and K321D, K321G, K321I, and K321T. Substitution of R251 and K256 to other amino acid residues generally resulted in increased activity on the amylose substrate.

Substitutions at positions N88, A252, A253, N308, A316, S357, T400, R402, and D403 resulted in no improvement in activity on the amylose substrate or a decrease in activity, while substitutions at positions N4, G5, T38, N93, G94, I95, Q96, V97, Y230, G255, V315, P319, A322, L354, T355, R356, G359, Y396, A397, Y398, G399, and Q401 resulted in reduced expression (referring to SEQ ID NO: 4).

## Claims

1. A method for producing a variant α-amylase polypeptide having altered binding to starch bundles, in which individual starch polymers are aligned, as compared to a parental α-amylase polypeptide, comprising:
introducing into the amino sequence of a parent Family 13 α-amylase polypeptide a mutation at an amino acid residue in the starch-binding groove that binds to starch bundles, wherein the mutation alters the binding of starch bundles to the variant α-amylase polypeptide compared to the parental α-amylase polypeptide;
wherein the starch-binding groove is formed by amino acid residues in the α-helix preceding the first β-stand in the A domain, the loop between the sixth α-helix and the seventh β-strand in the A domain, the loop between the seventh α-helix and the eighth β-strand in the A domain, and the loop connecting the A domain and the C domain;
the starch-binding groove corresponds to amino acid residues 1-6, 36, 38, 91-97, 224-226, 249-257, 278-282, 309-320, 354-359, 391, and 395-402, referring to SEQ ID NO: 2 for numbering, amino acid residues 1-6, 37, 39, 92-98, 227-229, 252-260, 281-285, 312-323, 357-362, 391, and 395-402, referring to SEQ ID NO: 3 for numbering, or amino acid residues 1-4, 36, 38, 91-97, 226-228, 251-259, 280-284, 311-322, 356-361, 363, 391, and 395-402, referring to SEQ ID NO: 4 for numbering;
wherein the parent has at least 80% amino acid sequence identity to SEQ ID NO: 4;
wherein the mutation is the substitution of the wild-type residue at a position corresponding to position 92 in SEQ ID NO: 4 with L, the substitution of the wild-type residue at a position corresponding to position 251 in SEQ ID NO: 4 with A, C, D, E, F, G, H, L, M, N, Q, S, T, or W, the substitution of the wild-type residue at a position corresponding to position 254 in SEQ ID NO: 4 with H, K, W, or Y, the substitution of the wild-type residue at a position corresponding to position 256 in SEQ ID NO: 4 with A, E, T, or V, the substitution of the wild-type residue at a position corresponding to position 317 in SEQ ID NO: 4 with C or R, the substitution of the wild-type residue at a position corresponding to position 318 in SEQ ID NO: 4 with A, F, H, K, Q, or R, the substitution of the wild-type residue at a position corresponding to position 320 in SEQ ID NO: 4 with A, H, M, N, or P, or the substitution of the wild-type residue at a position corresponding to position 321 in SEQ ID NO: 4 with D, G, I, or T; and
wherein the variant exhibits increased hydrolysis activity on an amylose substrate compared to the parent.

2. The method of claim 1, wherein the variant further comprises wild-type amino acid residues at one or more positions corresponding to N88, A252, A253, N308, A316, S357, T400, R402, and D403, referring to SEQ ID NO: 4 for numbering.

3. The method of claim 1 or claim 2, wherein the variant further comprises wild-type amino acid residues at one or more positions corresponding to N4, G5, T38, N93, G94, 195, Q96, V97, Y230, G255, V315, P319, A322, L354, T355, R356, G359, Y396, A397, Y398, G399, and Q401 in SEQ ID NO: 4, referring to SEQ ID NO: 4 for numbering.

4. The method of any one of the preceding claims, wherein the variant further comprises N at position 88, A at position 252, A at position 253, N at position 308, A at position 316, S at position 357, T at position 400, R at position 402, or D at position 403, referring to SEQ ID NO: 4 for numbering.

5. The method of any one of the preceding claims, wherein the variant further comprises N at position 4, G at position 5, T at position 38, N at position 93, G at position 94, I at position 95, Q at position 96, V at position 97, Y at position 230, G at position 255, V at position 315, P at position 319, A at position 322, L at position 354, T at position 355, R at position 356, G at position 359, Y at position 396, A at position 397, Y at position 398, G at position 399, and Q at position 401, referring to SEQ ID NO: 4 for numbering.

6. The method of any one of the preceding claims, wherein relative starch-binding is determined using cyclodextrin.

7. The method of any one of the preceding claims, wherein the parent has at least 90% amino acid sequence identity to SEQ ID NO: 4.

8. The method of any one of the preceding claims, wherein the variant exhibits improved starch liquefaction, starch saccharification, or cleaning performance compared to the parent.

## Patentansprüche

1. Verfahren zur Herstellung einer α-Amylasepolypeptid-Variante, die im Vergleich zu einem α-Amylaseausgangspolypeptid eine veränderte Bindung an Stärkebündel aufweist, in denen einzelne Stärkepolymere ausgerichtet sind, wobei das Verfahren Folgendes umfasst:
das Einführen einer Mutation in die Aminosäuresequenz eines α-Amylaseausgangspolypeptids der Familie 13 an einem Aminosäurerest in der stärkebindenden Furche, die an Stärkebündel bindet, wobei die Mutation die Bindung von Stärkebündeln an die α-Amylasepolypeptid-Variante im Vergleich zu dem α-Amylaseausgangspolypeptid ändert;
wobei die stärkebindende Furche von Aminosäureresten in der α-Helix gebildet wird, die dem ersten β-Strang in der A-Domäne, der Schleife zwischen der sechsten α-Helix und dem siebenten β-Strang in der A-Domäne, der Schleife zwischen der siebenten α-Helix und dem achten β-Strang in der A-Domäne und der Schleife, welche die A-Domäne mit der C-Domäne verbindet, vorangeht;
die stärkebindende Furche den Aminosäureresten 1-6, 36, 38, 91-97, 224-226, 249-257, 278-282, 309-320, 354-359, 391 und 395-402 von SEQ ID NO: 2, den Aminosäureresten 1-6, 37, 39, 92-98, 227-229, 252-260, 281-285, 312-323, 357-362, 391 und 395-402 von SEQ ID NO: 3 oder den Aminosäureresten 1-4, 36, 38, 91-97, 226-228, 251-259, 280-284, 311-322, 356-361, 363, 391 und 395-402 von SEQ ID NO: 4 entspricht;
wobei die Ausgangsverbindung zumindest 80 % Aminosäuresequenzidentität mit SEQ ID NO: 4 aufweist;
wobei die Mutation die Substitution des Wildtyp-Rests an einer Position, die Position 92 von SEQ ID NO: 4 entspricht, mit L, die Substitution des Wildtyp-Rests an einer Position, die Position 251 von SEQ ID NO: 4 entspricht, mit A, C, D, E, F, G, H, L, M, N, Q, S, T oder W, die Substitution des Wildtyp-Rests an einer Position, die Position 254 in SEQ ID NO: 4 entspricht, mit H, K, W oder Y, die Substitution des Wildtyp-Rests an einer Position, die Position 256 in SEQ ID NO: 4 entspricht, mit A, E, T oder V, die Substitution des Wildtyp-Rests an einer Position, die Position 317 in SEQ ID NO: 4 entspricht, mit C oder R, die Substitution des Wildtyp-Rests an einer Position, die Position 318 in SEQ ID NO: 4 entspricht, mit A, F, H, K, Q, oder R, die Substitution des Wildtyp-Rests an einer Position, die Position 320 in SEQ ID NO: 4 entspricht, mit A, H, M, N oder P oder die Substitution des Wildtyp-Rests an einer Position, die Position 321 in SEQ ID NO: 4 entspricht, mit D, G, I oder T darstellt; und
wobei die Variante eine erhöhte Hydrolyseaktivität auf einem Amylosesubstrat im Vergleich zur Ausgangsverbindung zeigt.

2. Verfahren nach Anspruch 1, wobei die Variante außerdem Wildtyp-Aminosäurereste an einer oder mehreren Positionen umfasst, die N88, A252, A253, N308, A316, S357, T400, R402 und D403 von SEQ ID NO: 4 entsprechen.

3. Verfahren nach Anspruch 1 oder 2, wobei die Variante außerdem Wildtyp-Aminosäurereste an einer oder mehreren Positionen umfasst, die N4, G5, T38, N93, G94, I95, Q96, V97, Y230, G255, V315, P319, A322, L354, T355, R356, G359, Y396, A397, Y398, G399 und Q401 von SEQ ID NO: 4 entsprechen.

4. Verfahren nach einem der vorangegangenen Ansprüche, wobei die Variante außerdem N an Position 88, A an Position 252, A an Position 253, N an Position 308, A an Position 316, S an Position 357, T an Position 400, R an Position 402 oder D an Position 403 von SEQ ID NO: 4 umfasst.

5. Verfahren nach einem der vorangegangenen Ansprüche, wobei die Variante außerdem N an Position 4, G an Position 5, T an Position 38, N an Position 93, G an Position 94, I an Position 95, Q an Position 96, V an Position 97, Y an Position 230, G an Position 255, V an Position 315, P an Position 319, A an Position 322, L an Position 354, T an Position 355, R an Position 356, G an Position 359, Y an Position 396, A an Position 397, Y an Position 398, G an Position 399 und Q an Position 401 von SEQ ID NO: 4 umfasst.

6. Verfahren nach einem der vorangegangenen Ansprüche, wobei die relative Stärkebindung unter Verwendung von Cyclodextrin bestimmt wird.

7. Verfahren nach einem der vorangegangenen Ansprüche, wobei die Ausgangsverbindung zumindest 90 % Aminosäuresequenzidentität mit SEQ ID NO: 4 aufweist.

8. Verfahren nach einem der vorangegangenen Ansprüche, wobei die Variante eine verbesserte Stärkeverflüssigung, Stärkeverzuckerung oder Reinigungsleistung im Vergleich zu der Ausgangsverbindung zeigt.

## Revendications

1. Procédé de production d'un polypeptide α-amylase variant ayant une liaison modifiée à des faisceaux d'amidon, dans lequel des polymères d'amidon individuels sont alignés, par comparaison à un polypeptide α-amylase parent, comprenant les étapes consistant à :
introduire dans la séquence amino d'un polypeptide α-amylase de la famille 13 parent une mutation au niveau d'un résidu d'acide aminé dans le sillon de liaison d'amidon qui se lie à des faisceaux d'amidon, dans lequel la mutation modifie la liaison de faisceaux d'amidon au polypeptide α-amylase variant par rapport au polypeptide α-amylase parent ;
dans lequel le sillon de liaison d'amidon est formé par des résidus d'acides aminés dans l'hélice α précédant le premier brin β dans le domaine A, la boucle entre la sixième hélice α et le septième brin β dans le domaine A, la boucle entre la septième hélice α et le huitième brin β dans le domaine A, et la boucle reliant le domaine A et le domaine C ;
le sillon de liaison d'amidon correspond aux résidus d'acides aminés 1-6, 36, 38, 91-97, 224-226, 249-257, 278-282, 309-320, 354-359, 391, et 395-402, en se référant à SEQ ID NO : 2 pour la numérotation, aux résidus d'acides aminés 1-6, 37, 39, 92-98, 227-229, 252-260, 281-285, 312-323, 357-362, 391 et 395-402, en se référant à SEQ ID NO : 3 pour la numérotation, ou aux résidus d'acides aminés 1-4, 36, 38, 91-97, 226-228, 251-259, 280-284, 311-322, 356-361, 363, 391 et 395-402, en se référant à SEQ ID NO : 4 pour la numérotation ;
dans lequel le parent a au moins 80 % d'identité de séquence d'acides aminés par rapport à SEQ ID NO : 4 ;
dans lequel la mutation est la substitution du résidu de type sauvage dans une position correspondant à la position 92 dans SEQ ID NO : 4 avec L, la substitution du résidu de type sauvage dans une position correspondant à la position 251 dans SEQ ID NO : 4 avec A, C, D, E, F, G, H, L, M, N, Q, S, T ou W, la substitution du résidu de type sauvage dans une position correspondant à la position 254 dans SEQ ID NO : 4 avec H, K, W ou Y, la substitution du résidu de type sauvage dans une position correspondant à la position 256 dans SEQ ID NO : 4 avec A, E, T ou V, la substitution du résidu sauvage dans une position correspondant à la position 317 dans SEQ ID NO : 4 avec C ou R, la substitution du résidu sauvage dans une position correspondant à la position 318 dans SEQ ID NO : 4 avec A, F, H, K, Q ou R, la substitution du résidu de type sauvage dans une position correspondant à la position 320 dans SEQ ID NO : 4 avec A, H, M, N ou P, ou la substitution du résidu de type sauvage dans une position correspondant à la position 321 dans SEQ ID NO : 4 avec D, G, I ou T; et
dans lequel le variant présente une activité d'hydrolyse accrue sur un substrat d'amylose par comparaison au parent.

2. Procédé selon l'une quelconque des revendications précédentes, dans lequel le variant comprend en outre des résidus d'acides aminés de type sauvage dans une ou plusieurs positions correspondant à N88, A252, A253, N308, A316, S357, T400, R402, et D403, en se référant à SEQ ID NO : 4 pour la numérotation.

3. Procédé selon l'une quelconque des revendications précédentes, dans lequel le variant comprend en outre des résidus d'acides aminés de type sauvage dans une ou plusieurs positions correspondant à N4, G5, T38, N93, G94, 195, Q96, V97, Y230, G255, V315, P319, A322, L354, T355, R356, G359, Y396, A397, Y398, G399, et Q401 dans SEQ ID NO : 4, en se référant à SEQ ID NO : 4 pour la numérotation.

4. Procédé selon l'une quelconque des revendications précédentes, dans lequel le variant comprend en outre N en position 88, A en position 252, A en position 253, N en position 308, A en position 316, S en position 357, T en position 400, R en position 402, ou D en position 403, en se référant à SEQ ID NO : 4 pour la numérotation.

5. Procédé selon l'une quelconque des revendications précédentes, dans lequel le variant comprend en outre N en position 4, G en position 5, T en position 38, N en position 93, G en position 94, I en position 95, Q en position 96, V en position 97, Y en position 230, G en position 255, V en position 315, P en position 319, A en position 322, L en position 354, T en position 355, R en position 356, G en position 359, Y en position 396, A en position 397, Y en position 398, G en position 399, et Q en position 401, en se référant à SEQ ID NO : 4 pour la numérotation.

6. Procédé selon l'une quelconque des revendications précédentes, dans lequel la liaison d'amidon est déterminée en utilisant de la cyclodextrine.

7. Procédé selon l'une quelconque des revendications précédentes, dans lequel le parent a au moins 90 % d'identité de séquence d'acides aminés avec SEQ ID NO : 4.

8. Procédé selon l'une quelconque des revendications précédentes, dans lequel le variant présente une liquéfaction d'amidon, une saccharification d'amidon ou une performance de nettoyage améliorées par comparaison au parent.
